# EUROPEAN PATENT SPECIFICATION

(11) **EP 1 732 609 B1**
(45) Date of publication and mention of the grant of the patent: **11.07.2012**
(21) Application number: 05729248.4
(22) Date of filing: 11.03.2005
(51) Int. Cl.: A61K 47/48, C07K 14/505, A61K 38/00

(54) **CONJUGATES OF HYDROXYALKYL STARCH AND A PROTEIN**
KONJUGATE VON HYDROXYALKYL-STÄRKE UND EINEM PROTEIN
CONJUGUÉS D'AMIDON HYDROXYALKYLIQUE ET D'UNE PROTÉINE

(30) Priority: 11.03.2004 EP 04005875; 11.03.2004 US 552069 P
(43) Date of publication of application: 20.12.2006
(60) Divisional application: 10011195.4
(73) Proprietor: Fresenius Kabi Deutschland GmbH, 61352 Bad Homburg v.d.H. (DE)
(72) Inventor: ZANDER, Norbert, 38527 Meine (DE); FRANK, Ronald, 38527 Meine Grassel (DE)
(74) Representative: Altmann, Andreas
(86) International application number: PCT/EP2005/002640
(87) International publication number: WO 2005/092391

(56) References cited:
- EP-A- 0 342 557
- EP-A- 1 398 327
- WO-A-01/70272
- WO-A-02/20033
- WO-A-02/080979
- WO-A-03/070772
- WO-A-03/074087
- WO-A-2004/024761
- WO-A-2005/014050
- WO-A-2005/014655
- WO-A-2005/074993
- WO-A-2005/092390
- STETSENKO D A ET AL: "EFFICIENT CONJUGATION OF PEPTIDES TO OLIGONUCLEOTIDES BY NATIVE LIGATION" JOURNAL OF ORGANIC CHEMISTRY, AMERICAN CHEMICAL SOCIETY. EASTON, US, vol. 65, 2000, pages 4900-4908, XP000992973 ISSN: 0022-3263
- DE KONING M C ET AL: "An approach to the synthesis of peptide-PNA-peptide conjugates via native ligation" TETRAHEDRON LETTERS, ELSEVIER, AMSTERDAM, NL, vol. 43, no. 45, 4 November 2002 (2002-11-04), pages 8173-8176, XP004387215 ISSN: 0040-4039

## Description

The present invention relates to conjugates of an active substance and hydroxyalkyl starch, preferably hydroxyethyl starch, wherein the conjugates are prepared by native chemical ligation via covalently linking the hydroxyalkyl starch and the active substance by reacting a thioester group with an alpha-X beta-amino group, wherein X is selected from the group consisting of -SH and 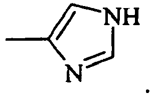 The present invention also relates to the method of producing these conjugates and the use of the conjugates.

Native chemical ligation is a highly effective method for preparing large peptides and small proteins where a peptide thioester is coupled with a peptide bearing an N-terminal cysteine residue to provide a product containing an amide bond at the site of the ligation.

WO 03/031581 A2 discloses a method of conjugating a polymer derivative to a polypeptide having a cysteine or histidine residue at the N-terminus, wherein said method comprises providing a polypeptide having a cysteine or histidine residue at the N-terminus, providing a thioester-terminated polymer, the polymer comprising a water soluble and non-peptidic polymer backbone, preferably a polyethylene glycole polymer, and reacting the polymer derivative and the polypeptide. As polymers, poly(alkylene glycol), copolymers of ethylene glycol and propylene glycol, poly(oxyethylated polyol), poly(olefinic alcohol), poly(vinylpyrrolidone), poly(alpha hydroxy acid), poly(vinyl alcohol), polyphosphazene, polyoxazoline, poly(N-acryloylmorpholine), polyacrylate, polyacrylamides, polysaccharides, and copolymers, terpolymers and mixtures thereof. All explicitly disclosed polymers are polyethylenglycol polymers.

WO 02/20033 describes native chemical ligation of polymer modified peptide fragments. Despite the progress of coupling methods and use of monofunctional PEG-molecules, a general disadvantage of PEGylated drugs is that the metabolization pathway of PEG as a non-natural polymer is not known in detail.

Therefore, it was an object of the present invention to provide novel conjugates of an active substance and a polymer formed by chemical ligation wherein no polyalkylene glycol, especially no polyethylene glycol is used as polymer.

Accordingly, it was another object of the present invention to provide a method for preparing these conjugates.

Thus, the present invention relates to a conjugate of an active substance and hydroxyalkyl starch, wherein the active substance and the hydroxyalkyl starch are linked by a chemical moiety, having a structure according to formula (IV) 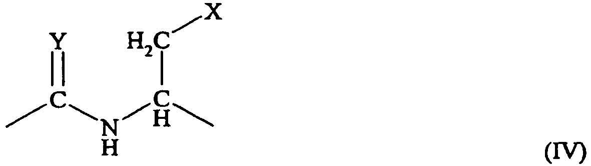 wherein Y is a heteroatom, selected from the group consisting of O and S, and X is selected from the group consisting of SH and 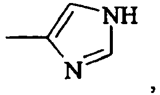 said conjugate having a structure according to formula (II) 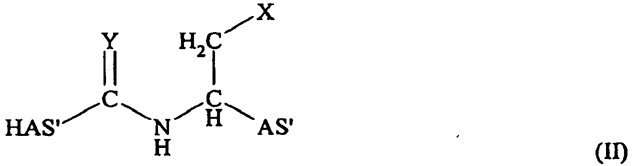 wherein HAS' is the residue of the hydroxyalkyl starch derivative which was linked to the thioester group by reacting the hydroxyalkyl starch with an at least bifunctional compound comprising a functional group M which is reacted with the hydroxyalkyl starch via the non-oxidized reducing end, and a functional group Q which is a thioester group or a functional group which is further modified to give a thioester group, to give the hydroxyalkyl starch derivative comprising said thioester group, and wherein AS' is the residue of the active substance or a derivative thereof which was linked to the alpha-X beta-amino group, and wherein the functional group M is selected from the group consisting of 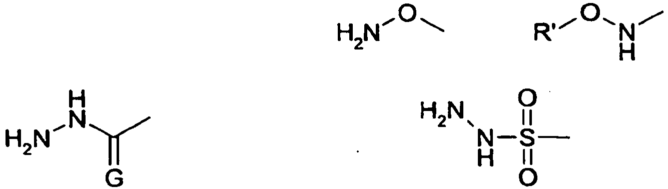 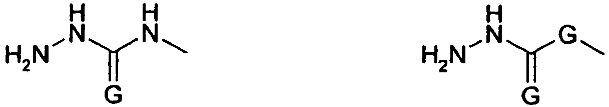 wherein G is O or S and, if present twice, independently O or S, and R' is methyl, and wherein the group -(C=Y) is derived from the thioester group -(C=Y)-S-R' and the group HN-CH-CH₂-X is derived from the alpha-X beta amino group.

Accordingly, the present invention relates to a method for preparing a covalently linked conjugate of an active substance and hydroxyalkyl starch, wherein the active substance and the hydroxyalkyl starch are linked by chemical residue having a structure according to formula (I) 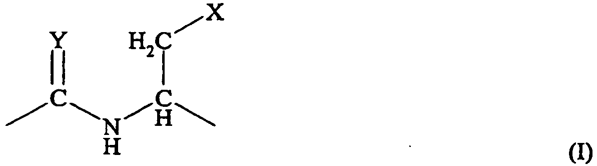 wherein Y is a heteroatom, selected from the group consisting of O and S, said method comprising
reacting a thioester group -(C=Y)-S-R' of a hydroxyalkyl starch derivative comprising said thioester group with an alpha-X beta amino group 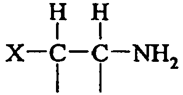 of an active substance derivative comprising said alpha-X beta amino group, said method further comprising
reacting a hydroxyalkyl starch with an at least bifunctional compound comprising a functional group M which is reacted with the hydroxyalkyl starch via the non-oxidized reducing end, and a functional group Q which is a thioester group or a functional group which is further modified to give a thioester group,
to give the hydroxyalkyl starch derivative comprising said thioester group, wherein R' is selected from the group consisting of hydrogen, an optionally suitably substituted, linear, cyclic and/or branched alkyl, aryl, heteroaryl, aralkyl, and heteroaralkyl group, preferably benzyl,
wherein X is selected from the group consisting of SH and 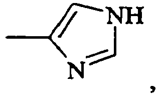 and wherein the group -(C=Y) is derived from the thioester group -(C=Y)-S-R' and the group HN-CH-CH₂-X is derived from the alpha-X beta amino group, and wherein the functional group M is selected from the group consisting of 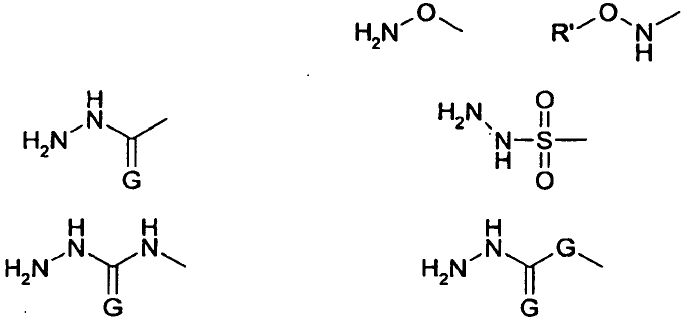 wherein G is O or S and, if present twice, independently O or S, and R' is methyl.

Therefore, the term "alpha-X beta-amino group" as used in the context of the present invention refers to an ethylene group in which X is bonded to a carbon atom and a primary amino group is bonded to the neighbouring carbon atom.

In the chemical moiety according to formula (I) above, the group -(C=Y) is derived from the thioester group -(C=Y)-S-R' and the group HN-CH-CH₂-X is derived from the alpha-X beta amino group.

The term "active substance" as used in the context of the present invention relates to a substance which can affect any physical or biochemical property of a biological organism including, but not limited to, viruses, bacteria, fungi, plants, animals, and humans. In particular, the term "active substance" as used in the context of the present invention relates to a substance intended for diagnosis, cure mitigation, treatment, or prevention of disease in humans or animals, or to otherwise enhance physical or mental well-being of humans or animals. Examples of biologically active molecules include, peptides, proteins, enzymes, small molecule drugs, dyes, lipids, nucleosides, oligonucleotides, polynucleotides, nucleic acids, cells, viruses, liposomes, microparticles, and micelles. Examples of proteins include, erythropoietin (EPO) such as recombinant human EPO (rhEPO), colony-stimulating factors (CSF), such as G-CSF like recombinant human G-CSF (rhG-CSF), alpha-Interferon (IFN alpha), beta-Interferon (IFN beta) or gamma-Interferon (IFN gamma), such as IFN alpha and IFN beta like recombinant human IFN alpha or IFN beta (rhIFN alpha or rhIFN beta), interleukines, e.g. IL-1 to IL-18 such as IL-2 or IL-3 like recombinant human IL-2 or IL-3 (rhIL-2 or rhIL-3), serum proteins such as coagulation factors II-XIII like factor VIII, alphal-antitrypsin (A1AT), activated protein C (APC), plasminogen activators such as tissue-type plasminogen activator (tPA), such as human tissue plasminogen activator (hTPA), AT III such as recombinant human AT III (rhAT III), myoglobin, albumin such as bovine serum albumin (BSA), growth factors, such as epidermal growth factor (EGF), thrombocyte growth factor (PDGF), fibroblast growth factor (FGF), brain-derived growth factor (BDGF), nerve growth factor (NGF), B-cell growth factor (BCGF), brain-derived neurotrophic growth factor (BDNF), ciliary neurotrophic factor (CNTF), transforming growth factors such as TGF alpha or TGF beta, BMP (bone morphogenic proteins), growth hormones such as human growth hormone, tumor necrosis factors such as TNF alpha or TNF beta, somatostatine, somatotropine, somatomedines, hemoglobin, hormones or prohormones such as insulin, gonadotropin, melanocyte-stimulating hormone (alpha-MSH), triptorelin, hypthalamic hormones such as antidiuretic hormones (ADH and oxytocin as well as releasing hormones and release-inhibiting hormones, parathyroid hormone, thyroid hormones such as thyroxine, thyrotropin, thyroliberin, prolactin, calcitonin, glucagon, glucagon-like peptides (GLP-1, GLP-2 etc.), exendines such as exendin-4, leptin, vasopressin, gastrin, secretin, integrins, glycoprotein hormones (e.g. LH, FSH etc.), melanoside-stimulating hormones, lipoproteins and apo-lipoproteins such as apo-B, apo-E, apo-Lₐ, immunoglobulins such as IgG, IgE, IgM, IgA, IgD and fragments thereof, hirudin, tissue-pathway inhibitor, plant proteins such as lectin or ricin, bee-venom, snake-venom, immunotoxins, antigen E, alpha-proteinase inhibitor, ragweed allergen, melanin, oligolysine proteins, RGD proteins or optionally corresponding receptors for one of these proteins; or a functional derivative or fragment of any of these proteins or receptors. Examples of enzymes include, carbohydrate-specific enzymes, proteolytic enzymes, oxidases, oxidoreductases, transferases, hydrolases, lyases, isomerases, kinases and ligases. Specific examples are asparaginase, arginase, arginin deaminase, adenosin deaminase, glutaminase, glutaminase-asparaginase, phenylalanin, tryptophanase, tyrosinase, superoxide dismutase (SOD), endotoxinase, catalase, peroxidase, kallikrein, trypsin, chymotrypsin, elastase, thermolysin, lipase, uricase, adenosine diphosphatase, purine nucleoside phosphorylase, bilirubin oxidase, glucose oxidase, glucodase, gluconate oxidase, galactosidase, glucocerebrosidase, glucuronidase, hyaluronidase, tissue factor, streptokinase, urokinase, MAP-kinases, DNAses, RNAses, lactoferrin and functional derivatives or fragments thereof.

According to one alternative of the present invention, the active substance is a small molecule drug, a peptide, and/or a protein.

Among others, the following proteins are to be mentioned explicitly: erythropoietin (EPO) such as recombinant human EPO (rhEPO), colony-stimulating factors (CSF), such as G-CSF like recombinant human G-CSF (rhG-CSF), serum proteins such as coagulation factors II-XIII like factor VII, factor VIII, or factor IX, alphal-antitrypsin (A1AT), activated protein C (APC), plasminogen activators such as tissue-type plasminogen activator (tPA), such as human tissue plasminogen activator (hTPA), AT III such as recombinant human AT III (rhAT III).

In the context of the present invention, the term "hydroxyalkyl starch" (HAS) refers to a starch derivative which has been substituted by at least one hydroxyalkyl group. A preferred hydroxyalkyl starch of the present invention has a constitution according to formula (II) 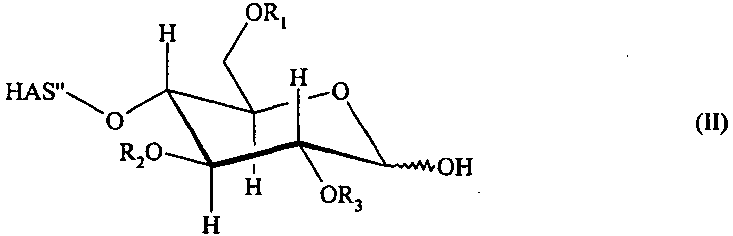 wherein the reducing end of the starch molecule is shown in the non-oxidized form and the terminal saccharide unit of HAS is shown in the acetal form which, depending on e.g. the solvent, may be in equilibrium with the aldehyde form. The abbreviation HAS" as used in the context of the present invention refers to the HAS molecule without the terminal saccharide unit at the reducing end of the HAS molecule.

The term hydroxyalkyl starch as used in the present invention is not limited to compounds where the terminal carbohydrate moiety comprises hydroxyalkyl groups R₁, R₂, and/or R₃ as depicted, for the sake of brevity, in formula (II), but also refers to compounds in which at least one hydroxyalkyl group is present anywhere, either in the terminal carbohydrate moiety and/or in the remaining part of the starch molecule, HAS", is substituted by a hydroxyalkyl group R₁, R₂, or R₃.

Hydroxyalkyl starch comprising two or more different hydroxyalkyl groups are also possible.

The at least one hydroxyalkyl group comprised in HAS may contain two or more hydroxy groups. According to a preferred embodiment, the at least one hydroxyalkyl group comprised HAS contains one hydroxy group.

The expression "hydroxyalkyl starch" also includes derivatives wherein the alkyl group is mono- or polysubstituted. In this context, it is preferred that the alkyl group is substituted with a halogen, especially fluorine, or with an aryl group. Furthermore, the hydroxy group of a hydroxyalkyl group may be esterified or etherified.

Furthermore, instead of alkyl, also linear or branched substituted or unsubstituted alkene groups may be used.

Hydroxyalkyl starch is an ether derivative of starch. Besides of said ether derivatives, also other starch derivatives can be used in the context of the present invention. For example, derivatives are useful which comprise esterified hydroxy groups. These derivatives may be e.g. derivatives of unsubstituted mono- or dicarboxylic acids with 2-12 carbon atoms or of substituted derivatives thereof. Especially useful are derivatives of unsubstituted monocarboxylic acids with 2-6 carbon atoms, especially derivatives of acetic acid. In this context, acetyl starch, butyryl starch and propionyl starch are preferred.

Furthermore, derivatives of unsubstituted dicarboxylic acids with 2-6 carbon atoms are preferred.

In the case of derivatives of dicarboxylic acids, it is useful that the second carboxy group of the dicarboxylic acid is also esterified. Furthermore, derivatives of monoalkyl esters of dicarboxylic acids are also suitable in the context of the present invention.

For the substituted mono- or dicarboxylic acids, the substitute groups may be preferably the same as mentioned above for substituted alkyl residues.

Techniques for the esterification of starch are known in the art (see e.g. Klemm D. et al, Comprehensive Cellulose Chemistry Vol. 2, 1998, Whiley-VCH, Weinheim, New York, especially chapter 4.4, Esterification of Cellulose (ISBN 3-527-29489-9).

According to a preferred embodiment of the present invention, hydroxyalkyl starch according to above-mentioned formula (II) is employed. The other saccharide ring structures comprised in HAS" may be the same as or different from the explicitly described saccharide ring.

As far as the residues R₁, R₂ and R₃ according to formula (II) are concerned there are no specific limitations. According to a preferred embodiment, R₁, R₂ and R₃ are independently hydrogen or a hydroxyalkyl group, a hydroxyaryl group, a hydroxyaralkyl group or a hydroxyalkaryl group having of from 2 to 10 carbon atoms in the respective alkyl residue or a group (CH₂CH₂O)ₙ-H, wherein n is an integer, preferably 1, 2, 3, 4, 5, or 6. Hydrogen and hydroxyalkyl groups having of from 2 to 10 are preferred. More preferably, the hydroxyalkyl group has from 2 to 6 carbon atoms, more preferably from 2 to 4 carbon atoms, and even more preferably from 2 to 4 carbon atoms. "Hydroxyalkyl starch" therefore preferably comprises hydroxyethyl starch, hydroxypropyl starch and hydroxybutyl starch, wherein hydroxyethyl starch and hydroxypropyl starch are particularly preferred and hydroxyethyl starch is most preferred.

The alkyl, aryl, aralkyl and/or alkaryl group may be linear or branched and suitably substituted.

Therefore, the present invention also relates to a method an a conjugate as described above wherein R₁, R₂ and R₃ are independently hydrogen or a linear or branched hydroxyalkyl group with from 2 to 6 carbon atoms.

Thus, R₁, R₂ and R₃ preferably may be hydroxyhexyl, hydroxypentyl, hydroxybutyl, hydroxypropyl such as 2-hydroxypropyl, 3-hydroxypropyl, 2-hydroxyisopropyl, hydroxyethyl such as 2-hydroxyethyl, hydrogen and the 2-hydroxyethyl group being especially preferred.

Therefore, the present invention also relates to a method and a conjugate as described above wherein R₁, R₂ and R₃ are independently hydrogen or a 2-hydroxyethyl group, an embodiment wherein at least one residue R₁, R₂ and R₃ being 2-hydroxyethyl being especially preferred.

Hydroxyethyl starch (HES) is most preferred for all embodiments of the present invention.

Therefore, the present invention relates to the method and the conjugate as described above, wherein the polymer is hydroxyethyl starch and the polymer derivative is a hydroxyethyl starch derivative.

Hydroxyethyl starch (HES) is a derivative of naturally occurring amylopectin and is degraded by alpha-amylase in the body. HES is a substituted derivative of the carbohydrate polymer amylopectin, which is present in corn starch at a concentration of up to 95 % by weight. HES exhibits advantageous biological properties and is used as a blood volume replacement agent and in hemodilution therapy in the clinics (Sommermeyer et al., 1987, Krankenhauspharmazie, 8(8), 271-278; and Weidler et al., 1991, Arzneim.-Forschung/Drug Res., 41, 494-498).

Amylopectin consists of glucose moieties, wherein in the main chain alpha-1,4-glycosidic bonds are present and at the branching sites alpha-1,6-glycosidic bonds are found. The physico-chemical properties of this molecule are mainly determined by the type of glycosidic bonds. Due to the nicked alpha-1,4-glycosidic bond, helical structures with about six glucose-monomers per turn are produced. The physico-chemical as well as the biochemical properties of the polymer can be modified via substitution. The introduction of a hydroxyethyl group can be achieved via alkaline hydroxyethylation. By adapting the reaction conditions it is possible to exploit the different reactivity of the respective hydroxy group in the unsubstituted glucose monomer with respect to a hydroxyethylation. Owing to this fact, the skilled person is able to influence the substitution pattern to a limited extent.

HES is mainly characterized by the molecular weight distribution and the degree of substitution. There are two possibilities of describing the substitution degree:
1. The degree of substitution can be described relatively to the portion of substituted glucose monomers with respect to all glucose moieties.
2. The degree of substitution can be described as the molar substitution, wherein the number of hydroxyethyl groups per glucose moiety are described.
In the context of the present invention, the degree of substitution, denoted as DS, relates to the molar substitution, as described above (see also Sommermeyer et al., 1987, Krankenhauspharmazie, 8(8), 271-278, as cited above, in particular p. 273).

HES solutions are present as polydisperse compositions, wherein each molecule differs from the other with respect to the polymerisation degree, the number and pattern of branching sites, and the substitution pattern. HES is therefore a mixture of compounds with different molecular weight. Consequently, a particular HES solution is determined by average molecular weight with the help of statistical means. In this context, Mₙ is calculated as the arithmetic mean depending on the number of molecules. Alternatively, M_{w} (or MW), the weight average molecular weight, represents a unit which depends on the mass of the HES.

In the context of the present invention, hydroxyethyl starch may preferably have a mean molecular weight (weight average molecular weight) of from 1 to 300 kD. Hydroxyethyl starch can further exhibit a preferred molar degree of substitution of from 0.1 to 0.8 and a preferred ratio between C₂ : C₆ substitution in the range of from 2 to 20 with respect to the hydroxyethyl groups.

The term "mean molecular weight" as used in the context of the present invention relates to the weight as determined according to the LALLS-(low angle laser light scattering)-GPC method as described in Sommermeyer et al., 1987, Krankenhauspharmazie, 8(8), 271-278; and Weidler et al., 1991, Arzneim.-Forschung/Drug Res., 41, 494-498. For mean molecular weights of 10 kD and smaller, additionally, the calibration was carried out with a standard which had previously been qualified by LALLS-GPC.

According to a preferred embodiment of the present invention, the mean molecular weight of hydroxyethyl starch employed is from 1 to 300 kD, more preferably from 2 to 200 kD, more preferably of from 4 to 130 kD, more preferably from 4 to 100 kD and still more preferably of from 4 to 70 kD.

Therefore, the present invention also relates to a method and to conjugates as described above wherein the hydroxyalkyl starch is hydroxyethyl starch having a mean molecular weight of from 4 to 70 kD.

Preferred ranges of the mean molecular weight are, e.g., 4 to 70 kD or 10 to 70 kD or 12 to 70 kD or 18 to 70 kD or 50 to 70 kD or 4 to 50 kD or 10 to 50 kD or 12 to 50 kD or 18 to 50 kD or 4 to 18 kD or 10 to 18 kD or 12 to 18 kD or 4 to 12 kD or 10 to 12 kD or 4 to 10 kD.

According to particularly preferred embodiments of the present invention, the mean molecular weight of hydroxyethyl starch employed is in the range of from more than 4 kD and below 70 kD, such as about 10 kD, or in the range of from 9 to 10 kD or from 10 to 11 kD or from 9 to 11 kD, or about 12 kD, or in the range of from 11 to 12 kD or from 12 to 13 kD or from 11 to 13 kD, or about 18 kD, or in the range of from 17 to 18 kD or from 18 to 19 kD or from 17 to 19 kD, or about 50 kD, or in the range of from 49 to 50 kD or from 50 to 51 kD or from 49 to 51 kD.

As to the upper limit of the molar degree of substitution (DS), values of up to 2.0 such as 0.9, 1.0, 1.1, 1.2, 1.3, 1.4, 1.5, 1.6, 1.7, 1.8, 1.9 or 2.0 are also possible, values of below 2.0 being preferred, values of below 1.5 being more preferred, values of below 1.0 such as 0.7, 0.8 or 0.9 being still more preferred.

Therefore, preferred ranges of the molar degree of substitution are from 0.1 to 2 or from 0.1 to 1.5 or from 0.1 to 1.0 or from 0.1 to 0.9 or from 0.1 to 0.8. More preferred ranges of the molar degree of substitution are from 0.2 to 2 or from 0.2 to 1.5 or from 0.2 to 1.0 or from 0.2 to 0.9 or from 0.2 to 0.8. Still more preferred ranges of the molar degree of substitution are from 0.3 to 2 or from 0.3 to 1.5 or from 0.3 to 1.0 or from 0.3 to 0.9 or from 0.3 to 0.8. Even more preferred ranges of the molar degree of substitution are from 0.4 to 2 or from 0.4 to 1.5 or from 0.4 to 1.0 or from 0.4 to 0.9 or from 0.4 to 0.8.

As far as the degree of substitution (DS) is concerned, DS is preferably at least 0.1, more preferably at least 0.2, more preferably at least 0.3 and more preferably at least 0.4. Preferred ranges of DS are from 0.1 to 0.8, more preferably from 0.2 to 0.8, more preferably from 0.3 to 0.8 and even more preferably from 0.4 to 0.8, still more preferably from 0.1 to 0.7, more preferably from 0.2 to 0.7, more preferably from 0.3 to 0.7 and more preferably from 0.4 to 0.7. Particularly preferred values of DS are, e.g., 0.1, 0.2, 0.3, 0.4, 0.5, 0.6, 0.7 or 0.8, with 0.2, 0.3, 0.4, 0.5, 0.6, 0.7 or 0.8 being more preferred, 0.3, 0.4, 0.5, 0.6, 0.7 or 0.8 being even more preferred, 0.4, 0.5, 0.6, 0.7 or 0.8 being still more preferred and, e.g. 0.4 and 0.7 being particularly preferred.

In the context of the present invention, a given value of the molar degree of substitution such as 0.8 may be the exact value or may be understood as being in a range of from 0.75 to 0.84. Therefore, for example, a given value of 0.1 may be the exact value of 0.1 or be in the range of from 0.05 to 0.14, a given value of 0.4 may be the exact value of 0.4 or in the range of from 0.35 to 0.44, or a given value of 0.7 may be the exact value of 0.7 or be in the range of from 0.65 to 0.74.

Particularly preferred combinations of molecular weight of the hydroxyalkyl starch, preferably hydroxyethyl starch, and its degree of substitution DS are, e.g., 10 kD and 0.4 or 10 kD and 0.7 or 12 kD and 0.4 or 12 kD and 0.7 or 18 kD and 0.4 or 18 kD and 0.7 or 50 kD and 0.4 or 50 kD and 0.7 or 100 kD and 0.7.

An example of HES having a mean molecular weight of about 130 kD is a HES with a degree of substitution of 0.2 to 0.8 such as 0.2, 0.3, 0.4, 0.5, 0.6, 0.7, or 0.8, preferably of 0.4 to 0.7 such as 0.4, 0.5, 0.6, or 0.7.

An example for HES with a mean molecular weight of about 130 kD is Voluven® from Fresenius. Voluven® is an artifical colloid, employed, e.g., for volume replacement used in the therapeutic indication for therapy and prophylaxis of hypovolaemia. The characteristics of Voluven® are a mean molecular weight of 130,000 +/- 20,000 D, a molar substitution of 0.4 and a C2 : C6 ratio of about 9:1.

As far as the ratio of C₂ : C₆ substitution is concerned, said substitution is preferably in the range of from 2 to 20, more preferably in the range of from 2 to 15 and even more preferably in the range of from 3 to 12.

According to a further embodiment of the present invention, also mixtures of hydroxyethyl starches may be employed having different mean molecular weights and/or different degrees of substitution and/or different ratios of C₂ : C₆ substitution. Therefore, mixtures of hydroxyethyl starches may be employed having different mean molecular weights and different degrees of substitution and different ratios of C₂ : C₆ substitution, or having different mean molecular weights and different degrees of substitution and the same or about the same ratio of C₂ : C₆ substitution, or having different mean molecular weights and the same or about the same degree of substitution and different ratios of C₂ : C₆ substitution, or having the same or about the same mean molecular weight and different degrees of substitution and different ratios of C₂ : C₆ substitution, or having different mean molecular weights and the same or about the same degree of substitution and the same or about the same ratio of C₂ : C₆ substitution, or having the same or about the same mean molecular weights and different degrees of substitution and the same or about the same ratio of C₂ : C₆ substitution, or having the same or about the same mean molecular weight and the same or about the same degree of substitution and different ratios of C₂ : C₆ substitution, or having about the same mean molecular weight and about the same degree of substitution and about the same ratio of C₂ : C₆ substitution.

In different conjugates and/or different methods according to the present invention, different hydroxyalkyl starches, preferably different hydroxyethyl starches and/or different hydroxyalkyl starch mixtures, preferably different hydroxyethyl starch mixtures, may be employed.

In the context of the present invention, the term "reactive carboxy group" refers to a reactive ester, a carboxylic acid anhydride, isothiocyanates or isocyanate may be mentioned. Preferred reactive esters are derived from N-hydroxy succinimides such as N-hydroxy succinimide or Sulfo-N-hydroxy succinimide, suitably substituted phenols such as p-nitrophenol, o,p-dinitrophenol, o,o'-dinitrophenol, trichlorophenol such as 2,4,6-trichlorophenol or 2,4,5-trichlorophenol, trifluorophenol such as 2,4,6-trifluorophenol or 2,4,5-trifluorophenol, pentachlorophenol, pentafluorophenol, or hydroxyazoles such as hydroxy benzotriazole. Especially preferred are N-hydroxy succinimides, with N-hydroxy succinimide and Sulfo-N-hydroxy succinimide being especially preferred. All alcohols may be employed alone or as suitable combination of two or more thereof. As reactive ester, pentafluorophenyl ester and N-hydroxy succinimide ester are especially preferred.

As to the group R' of the thioester group -(C=Y)-S-R', preferably -(C=O)-S-R', no specific limitations exist given that the group -S-R' forms an electrophilic leaving group which is suitable for a displacement when reacted with the alpha-X beta-amino group. Preferred residues SR' include, substituents which are derived from substituted or unsubstituted thiophenol, thiopyridine, benzyl mercaptane, ethanethiol, methanethiol, 2-mercaptoethansulfonic acid, 2-mercaptoacetic acid, 2-mercaptoacetic acid methyl or ethyl ester, 3-mercaptopropionic acid, 3-mercaptopropionic acid methyl or ethyl ester, 4-mercaptobutyric acid, 4-mercaptobutyric acid methyl or ethyl ester.

According to the present invention, the thioester group is comprised in the hydroxyalkyl starch and the alpha-X beta-amino group is comprised in the active substance.

Therefore, the present invention also relates to a method as described above, wherein a thioester functionalized hydroxyalkyl starch is reacted with an alpha-X beta-amino group of the active substance.

The thioester functionalized hydroxyalkyl starch, preferably hydroxyethyl starch, is provided by reacting a hydroxyalkyl starch with an at least bifunctional compound comprising a functional group M which is reacted with the hydroxyalkyl starch via the non-oxidized reducing end, and a functional group Q which is a thioester group or a functional group which is further modified to give a thioester group.

Further a method which comprises selectively oxidizing hydroxyalkyl starch at its reducing end to give hydroxalkyl starch according to formula (IIIa) 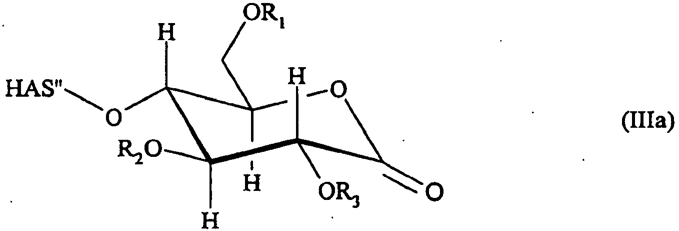 and/or according to formula (IIIb) 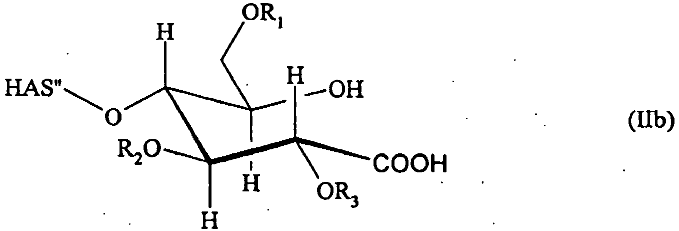 and reacting hydroxyalkyl starch selectively oxidized at its reducing end with at least one suitable compound to give a thioester functionalized Hydroxyalkyl starch is described.

Oxidation of the hydroxyalkyl starch, preferably hydroxyethyl starch, may be carried out according to each method or combination of methods which result in compounds having the above-mentioned structures (IIIa) and/or (IIIb). Although the oxidation may be carried out according to all suitable method or methods resulting in the oxidized reducing end of hydroxyalkyl starch, it is preferably carried out using an alkaline iodine solution as described, e.g., in DE 196 28 705 A1 the respective contents of which (example A, column 9, lines 6 to 24) is incorporated herein by reference.

According to the present invention, the hydroxyalkyl starch is reacted with an at least bifunctional compound comprising a functional group M which is reacted with the hydroxyalkyl starch and a functional group Q which is a thioester group or a functional group which can be modified to give a thioester group.

As functional group M of the at least bifunctional compound which is reacted with the hydroxyalkyl starch, (a group is described having the structure R'-NH-where R' is hydrogen or a alkyl, cycloalkyl, aryl, aralkyl, arylcycloalkyl, alkaryl or cycloalkylaryl residue where the cycloalkyl, aryl, aralkyl, arylcycloalkyl, alkaryl or cycloalkylaryl residue may be linked directly to the NH group or, according to another embodiment, may be linked by an oxygen bridge to the NH group. The alkyl, cycloalkyl, aryl, aralkyl, arylcycloalkyl, alkaryl, or cycloalkylaryl residues may be suitably substituted. As preferred substituents, halogens such as F, Cl or Br may be mentioned. Especially preferred residues R' are hydrogen, alkyl and alkoxy groups, and even more preferred are hydrogen and unsubstituted alkyl and alkoxy groups.

Among the alkyl and alkoxy groups, groups with 1, 2, 3, 4, 5, or 6 C atoms are preferred. More preferred are methyl, ethyl, propyl, isopropyl, methoxy, ethoxy, propoxy, and isopropoxy groups. Especially preferred are methyl, ethyl, methoxy, ethoxy, and particular preference is given to methyl or methoxy.

Further, a functional group M having the structure R'-NH-R"- is described where R" preferably comprises the structure unit -NH- and/or the structure unit -(C=G)- where G is O or S, and/or the structure unit -SO₂-. Specific examples for the functional group R" are 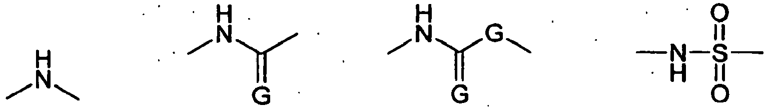 and 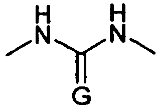 where, if G is present twice, it is independently O or S.

According to the invention, the functional group M is selected from the group consisting of 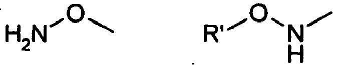 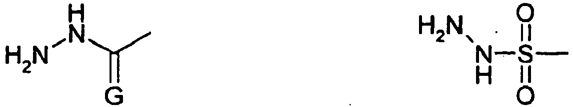 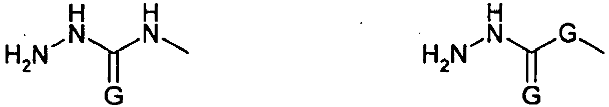 wherein G is O or S and, if present twice, independently O or S, and R' is methyl.

According to the group Q which is a thioester group or a functional group which can be chemically modified to give a thioester group, the following functional groups are to be mentioned, among others:
- C-C-double bonds or C-C-triple bonds or aromatic C-C-bonds;
- the thio group or the hydroxy groups;
- alkyl sulfonic acid hydrazide, aryl sulfonic acid hydrazide;
- 1,2-dioles;
- 1,2 amino-thioalcohols;
- azides;
- 1,2-aminoalcohols;
- the amino group -NH₂ or derivatives of the amino groups comprising the structure unit -NH- such as aminoalkyl groups, aminoaryl group, aminoaralkyl groups, or alkarlyaminogroups;
- the hydroxylamino group -O-NH₂, or derivatives of the hydroxylamino group comprising the structure unit -O-NH-, such as hydroxylalkylamino groups, hydroxylarylamino groups, hydroxylaralkylamino groups, or hydroxalalkarylamino groups;
- alkoxyamino groups, aryloxyamino groups, aralkyloxyamino groups, or alkaryloxyamino groups, each comprising the structure unit -NH-O-;
- residues having a carbonyl group, -Q-C(=G)-M, wherein G is O or S, and M is, for example,
   -- -OH or -SH;
   -- an alkoxy group, an aryloxy group, an aralkyloxy group, or an alkaryloxy group;
   -- an alkylthio group, an arylthio group, an aralkylthio group, or an alkarylthio group;
   -- an alkylcarbonyloxy group, an arylcarbonyloxy group, an aralkylcarbonyloxy group, an alkarylcarbonyloxy group;
   -- activated esters such as esters of hydroxylamines having imid structure such as N-hydroxysuccinimide or having a structure unit O-N where N is part of a heteroaryl compound or, with G = O and Q absent, such as aryloxy compounds with a substituted aryl residue such as pentafluorophenyl, paranitrophenyl or trichlorophenyl;
   wherein Q is absent or NH or a heteroatom such as S or O;
- -NH-NH₂, or -NH-NH-;
- -NO₂;
- the nitril group;
- carbonyl groups such as the aldehyde group or the keto group;
- the carboxy group;
- the -N=C=O group or the -N=C=S group;
- vinyl halide groups such as the vinyl iodide or the vinyl bromide group or triflate;
- -C≡C-H;
- -(C=NH₂Cl)-OAlkyl
- groups -(C=O)-CH₂-Hal wherein Hal is Cl, Br, or I;
- -CH=CH-SO₂-;
- a disulfide group comprising the structure -S-S-;
- the group 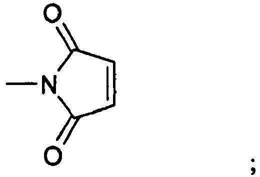
- the group

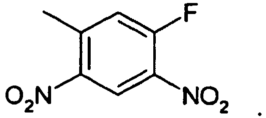

According to a first alternative, the functional group Q is a thioester group.

According to a second alternative, the functional group Q is a group which is further modified to give a thioester group. According to this embodiment, the hydroxyalkyl starch derivative resulting from the reaction of hydroxyalkyl starch, preferably selectively oxidized at its reducing end, with the at least bifunctional compound, is reacted with a further at least bifunctional compound comprising a functional group which is reacted with the functional group Q of the hydroxyalkyl starch derivative and a thioester group.

As functional group of the further compound which is reacted with the functional group Q, every suitable functional group from the list of functional groups described above for functional Q is to be mentioned given that it can be reacted with Q.

According to one embodiment of the present invention, the functional group Q comprises the chemical structure -NH-.

According to a preferred embodiment of the present invention, the functional group Q is a group having the structure R'-NH- where R' is hydrogen or a alkyl, cycloalkyl, aryl, aralkyl, arylcycloalkyl, alkaryl or cycloalkylaryl residue where the cycloalkyl, aryl, aralkyl, arylcycloalkyl, alkaryl or cycloalkylaryl residue may be linked directly to the NH group or, according to another embodiment, may be linked by an oxygen bridge to the NH group. The alkyl, cycloalkyl, aryl, aralkyl, arylcycloalkyl, alkaryl, or cycloalkylaryl residues may be suitably substituted. As preferred substituents, halogenes such as F, Cl or Br may be mentioned. Especially preferred residues R' are hydrogen, alkyl and alkoxy groups, and even more preferred are hydrogen and unsubstituted alkyl and alkoxy groups.

Among the alkyl and alkoxy groups, groups with 1, 2, 3, 4, 5, or 6 C atoms are preferred. More preferred are methyl, ethyl, propyl, isopropyl, methoxy, ethoxy, propoxy, and isopropoxy groups. Especially preferred are methyl, ethyl, methoxy, ethoxy, and particular preference is given to methyl or methoxy.

According to another embodiment of the present invention, the functional group Q has the structure R'-NH-R"- where R" preferably comprises the structure unit -NH- and/or the structure unit -(C=G)- where G is O or S, and/or the structure unit -SO₂-. According to more preferred embodiments, the functional group R" is selected from the group consisting of 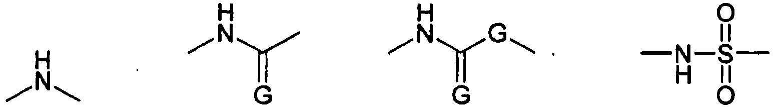 And 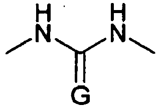 where, if G is present twice, it is independently O or S.

Therefore, the present invention also relates to a method and a conjugate as mentioned above wherein the functional group Q is selected from the group consisting of

H₂N-

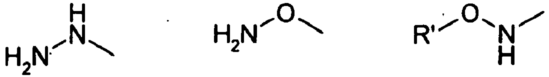

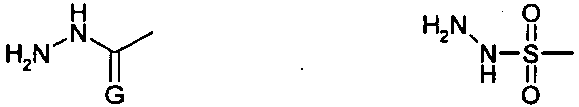

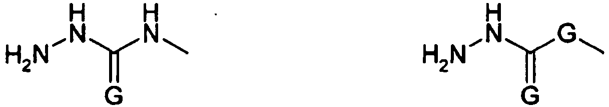

wherein G is O or S and, if present twice, independently O or S, and R' is methyl.

According to a particularly preferred embodiment of the present invention, the functional group Q is an amino group -NH₂.

According to one embodiment of the present invention, both M and Q comprise an amino group -NH-. Further, an embodiment is described wherein both M and Q are an amino group - NH₂.

According to a preferred embodiment of the present invention, the compound comprising M and Q is a homobifunctional compound, more preferably a homobifunctional compound comprising, as functional groups M and Q, most preferably the amino group -NH₂, or according to other embodiments, the hydroxylamino group -O-NH₂ or the group 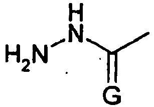 with G preferably being O. Specific examples for these compounds comprising M and Q are 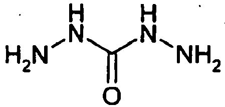 or 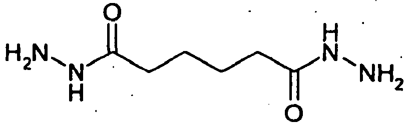 or

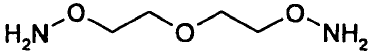

In the described case wherein both M and Q are an amino group -NH₂, M and Q may be separated by any suitable spacer. Among others, the spacer may be an optionally substituted, linear, branched and/or cyclic hydrocarbon residue. Generally, the hydrocarbon residue has from 1 to 60, preferably from 1 to 40, more preferably from 1 to 20, more preferably from 2 to 10, more preferably from 2 to 6 and especially preferably from 2 to 4 carbon atoms. If heteroatoms are present, the separating group comprises generally from 1 to 20, preferably from 1 to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group. According to an even more preferred embodiment, the hydrocarbon residue is an alkyl chain of from 1 to 20, preferably from 2 to 10, more preferably from 2 to 6, and especially preferably from 2 to 4 carbon atoms.

Therefore, a method and a conjugate as described above are described wherein the hydroxyalkyl starch is reacted with 1,4-diaminobutane, 1,3-diaminopropane or 1,2-diaminoethane to give an amino functionalized hydroxyalkyl starch derivative.

According to the present invention, the hydroxyalkyl starch is employed with non-oxidized reducing end. According to the present invention, the hydroxyalkyl starch is reacted with functional group M via the non-oxidized reducing end. The hydroxyalkyl starch may be reacted with functional group M at the non-oxidized reducing end and at least one further chemical moiety of the HAS molecule

The term "the hydroxyalkyl starch is reacted via the reducing end" or "the hydroxyalkyl starch is reacted via the selectively oxidized reducing end" as used in the context of the present invention relates to a process according to which the hydroxyalkyl starch is reacted predominantly via its (selectively oxidized) reducing end.

This term "predominantly via its (selectively oxidized) reducing end" relates to processes according to which statistically more than 50 %, preferably at least 55 %, more preferably at least 60 %, more preferably at least 65 %, more preferably at least 70 %, more preferably at least 75 %, more preferably at least 80 %, more preferably at least 85 %, more preferably at least 90 %, and still more preferably at least 95 % such as 95 %, 96 %, 97 %, 98 %, or 99 % of the hydroxyalkyl starch molecules employed for a given reaction are reacted via at least one (selectively oxidized) reducing end per hydroxyalkyl starch molecule, wherein a given hydroxyalkyl starch molecule which is reacted via at least one reducing end can be reacted in the same given reaction via at least one further suitable functional group which is comprised in said hydroxyalkyl starch molecule and which is not a reducing end. If one or more hydroxyalkyl starch molecule(s) is (are) reacted via at least one reducing and simultaneously via at least one further suitable functional group which is comprised in this (these) hydroxyalkyl starch molecule(s) and which is not a reducing end, statistically preferably more than 50 %, preferably at least 55 %, more preferably at least 60 %, more preferably at least 65 %, more preferably at least 70 %, more preferably at least 75 %, more preferably at least 80 %, more preferably at least 85 %, more preferably at least 90 %, and still more preferably at least 95 % such as 95 %, 96 %, 97 %, 98 %, or 99 % of all reacted functional groups of these hydroxyalkyl starch molecules, said functional groups including the reducing ends, are reducing ends.

The term "reducing end" as used in the context of the present invention relates to the terminal aldehyde group of a hydroxyalkyl starch molecule which may be present as aldehyde group and/or as corresponding acetal from. In case the reducing end is oxidized, the aldehyde or acetal group is in the form of a carboxy group and/or of the corresponding lactone.

As described above, the amino functionalized hydroxyalkyl starch derivative is preferably further reacted with an at least bifunctional compound comprising a functional group which is reacted with the amino group of the amino functionalized hydroxyalkyl starch derivative, and a thioester group.

As preferred compounds to be reacted with the amino functionalized hydroxyalkyl starch derivative, compounds are to be mentioned which comprise a reactive carboxy group which is reacted with the amino group, and a thioester group. As reactive carboxy group, a reactive ester, isothiocyanates or isocyanate may be mentioned. Preferred reactive esters are derived from N-hydroxy succinimides such as N-hydroxy succinimide or Sulfo-N-hydroxy succinimide, suitably substituted phenols such as p-nitrophenol, o,p-dinitrophenol, o,o'-dinitrophenol, trichlorophenol such as 2,4,6-trichlorophenol or 2,4,5-trichlorophenol, trifluorophenol such as 2,4,6-trifluorophenol or 2,4,5-trifluorophenol, pentachlorophenol, pentafluorophenol, or hydroxyazoles such as hydroxy benzotriazole.

The reactive carboxy group and the thioester may be separated by any suitable spacer. Among others, the spacer may be an optionally substituted, linear, branched and/or cyclic hydrocarbon residue. Generally, the hydrocarbon residue has from 1 to 60, preferably from 1 to 40, more preferably from 1 to 20, more preferably from 2 to 10, more preferably from 2 to 6 and especially preferably from 2 to 4 carbon atoms. If heteroatoms are present, the separating group comprises generally from 1 to 20, preferably from 1 to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group. According to one embodiment of the present invention, the reactive carboxy group and the thioester group are separated by an alkyl residue with 2, 3, or 4 carbon atoms such as an ethylen group, propylene group, or butylene group.

As an example, in case hydroxyalkyl starch, preferably hydroxyethyl starch, is reacted with its oxidized reducing end with a diaminoalkane such as 1,4-diaminobutane, 1,3-diaminopropane, or 1,2-diaminoethane, and the resulting hydroxyalkyl starch derivative is further reacted with a compound comprising a thioester group -S-R' and a reactive carboxy group where the thioester group and the reactive carboxy group are separated by a ethylen group, propylene group, or butylene group, a thioester functionalized hydroxyalkyl starch according to 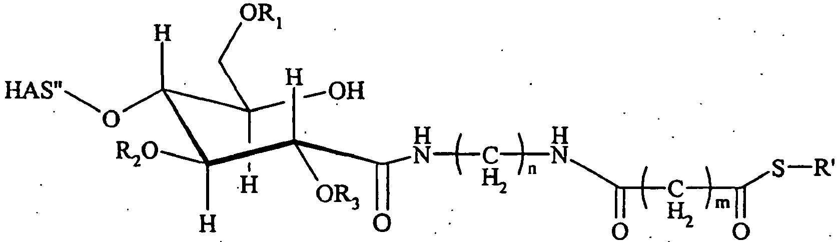 may result, where n, m are independently 2, 3, 4.

Therefore, also a method and a conjugate as described above are described said method comprising oxidizing hydroxyalkyl starch at its reducing end, reacting the oxidized reducing end with an at least bifunctional compound comprising two amino groups to give an amino functionalized hydroxyalkyl starch derivative, and reacting the amino group of the derivative with an at least bifunctional compound comprising at least one functional group which is reacted with the amino group of the derivative, preferably a reactive carboxy group, and comprising at least one thioester group, preferably one thioester group -SR'.

Further described is a thioester functionalized hydroxyalkyl starch which is prepared by selectively oxidizing hydroxyalkyl starch at its reducing end, as described above, and
- by converting the oxidized reducing end of the hydroxyalkyl starch to an imidazolide of a carboxylic acid, prepared, e.g., by reaction of the corresponding carboxylic acid with, e.g., N,N-carbonyldiimidazole) with a comparatively acidic thiol (Masamune, S., et al., J. Am. Chem. Soc. 98 (1976) 7874), or
- by converting the oxidized reducing end of the hydroxyalkyl starch using a disulfide and triphenylphosphine to the corresponding thioester (Mukaiyama, T., et al., Bull. Chem. Soc. Jpn. 43 (1970) 1271), or
- reacting the oxidized reducing end of the hydroxyalkyl starch with aryl thioisocyanates (Grieco, P., et al., Tetrahedron Lett. 43 (1979) 1283), or
- reacting the oxidized reducing end of the hydroxyalkyl starch with thiopyridyl chloroformate (Corey, E.J., et al., Tetrahedron Lett. (1979), 2875), or
- reacting the oxidized reducing end of the hydroxyalkyl starch with 2-fluoro-N-methylpyridinium tosylate and thiols (Watanabe, Y., et al., Chem. Lett. (1976) 741), or
- reacting the oxidized reducing end of the hydroxyalkyl starch with a carbodiimide such as such as diisopropyl carbodiimde (DIC), dicyclohexyl carbodiimides (DCC), 1-ethyl-3-(3-dimethylaminopropyl) carbodiimide (EDC), on a solid support immobilized EDC and a thiol. (C.E-Lin et al. Tetrahedron Lett. 43 (2002) 4531-34; M. Adamczyk, Tetrahedron Lett. 37 (1996) 4305-8, J. Hovinen, Nucleosides Nucleotides 18 (1999) 1263-4).

Therefore, also a method and a conjugate as described above, said method comprising oxidizing hydroxyalkyl starch at its reducing end and reacting the oxidized reducing end with carbodiimide such as such as diisopropyl carbodiimde (DIC), dicyclohexyl carbodiimides (DCC), 1-ethyl-3-(3-dimethylaminopropyl) carbodiimide (EDC), 1-ethyl-3-(3-dimethylaminopropyl) carbodiimide (EDC) being preferred and a thiol to give the thioester functionalized hydroxyalkyl starch, are described.

The thioester functionalized hydroxyalkyl starch derivative as described above is then reacted with the alpha-X beta-amino group of the active substance.

Therefore, the present invention also relates to a conjugate as described above according to formula (IV) 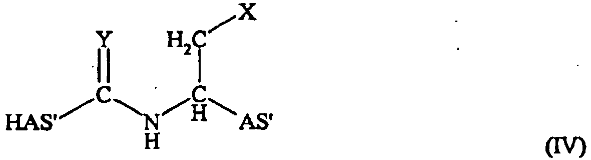 wherein HAS' is the residue of the hydroxyalkyl starch or derivative thereof which was linked to the thioester group, and wherein AS' is the residue of the active substance or a derivative thereof which was linked to the alpha-X beta-amino group.

According to a preferred embodiment of the present invention, the alpha-X beta-amino group is comprised in a cysteine residue or a histidine residue of the active substance, preferably of the small molecule drug, peptide, or protein.

Therefore, the present invention also relates to a method and a conjugate as described above, wherein the alpha-X beta-amino group is comprised in a cysteine residue or a histidine residue of the active substance.

More preferably, the cysteine or histidine residue of the active substance is an N-terminal cysteine or histidine of a peptide or a proteine. The N-terminal cysteine or histidine residue can be a naturally occuring N-terminal cysteine or histidine residue or can be N-terminally introduced in the peptide or protein by a suitable modification of the peptide or protein sequence. In peptides, the mentioned amino acids can be introduced during synthesis. Peptides synthesis is know in the art. (W. Chang, P. D. White ; Fmoc solid phase peptide synthesis, a practical approach; Oxford University Press, Oxford, 2000, ISBN 0199637245).

Recombinant polypeptides are obtainable by way of standard molecular biological techniques as, for example, described in Molecular Cloning: A Laboratory Manual, 3rd edition, Eds. Sambrook et al., CSHL Press 2001. Briefly, polypeptides can be expressed from recombinant expression vectors comprising a nucleic acid encoding the desired polypeptide, which nucleic acid is operably linked to at least one regulator sequence allowing expression of the desired polypeptide. For example, a nucleic acid sequence encoding the desired polypeptide can be isolated and cloned into an expression vector and the vector can then be transformed into a suitable host cell for expression of the desired polypeptide. Such a vector can be a plasmid, a phagemid or a cosmid. For example, a nucleic acid molecule can be cloned in a suitable fashion into prokaryotic or eukaryotic expression vectors *(Molecular Cloning,* see above). Such expression vectors comprise at least one promoter and can also comprise a signal for translation initiation and - in the case of prokaryotic expression vectors - a signal for translation termination, while in the case of eukaryotic expression vectors preferably expression signals for transcriptional termination and for polyadenylation are comprised. Examples for prokaryotic expression vectors are, for expression in Escherichia coli e.g. expression vectors based on promoters recognized by T7 RNA polymerase as described in US 4,952,496, for eukaryotic expression vectors for expression in Saccharomyces cerevisiae, e.g. the vectors G426/Met25 or P526/Gall (Mumberg et al., (1994) Nucl. Acids Res., 22, 5767-5768), for the expression in insect cells, e.g. Baculovirus vectors, as e.g described in EP-B1-0127839 or EP-B1-0549721 or by Ciccarone et al. ("Generation of recombinant Baculovirus DNA in E.coli using baculovirus shuttle vector" (1997) Volume 13, U. Reischt, ed. (Totowa, NJ: Humana Press Inc.) and for the expression in mammalian cells, e.g. the vectors Rc/CMW and Rc/ASW and SW40-Vectors, which are commonly known and commercially available, or the EBNA-system described in Example 4, the Sindbis replicon-based pCytTS (Boorsma et al. (2002) Biotechnol. Bioeng. 79(6): 602-609), the Sindbis virus expression system (Schlesinger (1993) Trends Biotechnol. 11(1):18-22) or an Adenovirus expression system (He et al. (1998) Proc. Natl. Acad. Sci. USA 95:2509-2514). The molecular biological methods for the production of these expression vectors as well as the methods for transfecting host cells and culturing such transfected host cells as well as the conditions for producing and obtaining the polypeptides of the invention from said transformed host cells are well known to the skilled person.

Polypeptides with the desired N-terminal Cys or His residue can be generated from the polypeptides expressed and purified as described above by cloning the polypeptide of interest behind an N-terminal leader sequence which is removable to yield the polypeptides with the desired N-terminal Cys or His residue.

This can be achieved, for example, by proteolytic cleavage of the polypeptide expressed and purified as described above. In such a case, a fusion polypeptide was cloned, expressed and purified wherein a Cys or His residue follows directly a highly selective protease cleavage site, for example a His or Cys residue immediately following the Factor Xa cleavage site: Ile (Glu/Asp) Gly Arg | (Cys/His), or a His or Cys residue immediately following the Enterokinase cleavage site: Asp Asp Asp Asp Lys | (Cys/His), wherein | denotes the site of cleavage by the protease.

This can further be achieved, for example, by cleavage of the polypeptide during expression, for example cleavage at the stage of ER translocation by the signal peptidase. In such a case, a fusion polypeptide was cloned and expressed wherein a Cys or His residue follows directly the signal peptide directing the recombinant polypeptide to the secretory pathway (for review see Rapoport et al., Annu Rev Biochem. 1996;65:271-303).

The molecular biological methods to manipulate the coding sequence of a recombinant polypeptide so that a coding sequence for a polypeptide with the desired Cys or His residue at the desired position is generated are well known in the art (Sambrook, above).

According to the native chemical ligation reaction according to the present invention, the intermediate product of the reaction of the thioester functionalized hydroxyalkyl starch and the alpha-X beta-amino functionalized active substance, preferably a peptide or protein with a cysteine or histidine residue, preferably a N-terminal cysteine or histidine residue, is a thioester which is irreversibly converted by intramolecular trans-acetylation to the inventive conjugate comprising the hydroxyalkyl starch derivative linked via an amide linkage to the active substance.

Further, a method as described above is described, wherein a thioester functionalized active substance is reacted with an alpha-X beta-amino group of a hydroxyalkyl starch derivative.

Accordingly, also a conjugate as described above is described, having a structure according to formula (V) 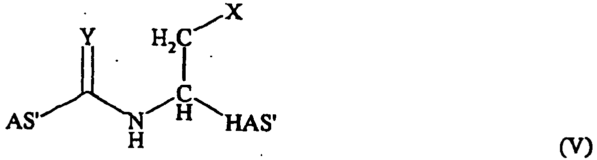 wherein HAS' is the residue of the hydroxyalkyl starch or derivative thereof which was linked to the alpha-X beta-amino group, and wherein AS' is the residue of the active substance or a derivative thereof which was linked to the thioester group.

The alpha-X beta-amino functionalized hydroxyalkyl starch can be prepared by any suitable method. According to one embodiment the alpha-X beta-amino functionalized hydroxyalkyl starch is prepared by a method comprising selectively oxidizing the hydroxyalkyl starch at its reducing end and suitably chemically modifying the oxidized reducing end to give an alpha-X beta-amino functionalized hydroxyalkyl starch derivative.

Therefore, according to one embodiment a method is described comprising selectively oxidizing hydroxyalkyl starch at its reducing end to give hydroxalkyl starch according to formula to formula (IIIa) 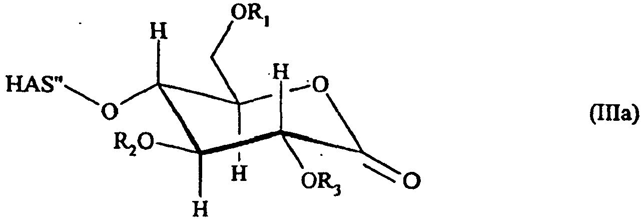 and/or according to formula (IIIb) 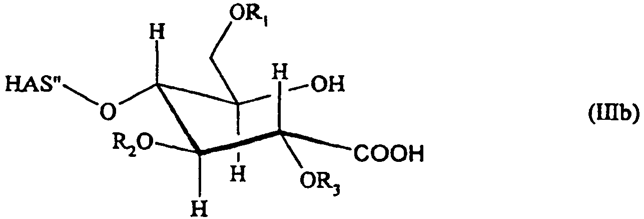 and suitably reacting hydroxyalkyl starch selectively oxidized at its reducing end to give a thioester functionalized hydroxyalkyl starch.

Oxidation of the hydroxyalkyl starch, preferably hydroxyethyl starch, may be carried out according to each method or combination of methods which result in compounds having the above-mentioned structures (IIIa) and/or (IIIb). Although the oxidation may be carried out according to all suitable method or methods resulting in the oxidized reducing end of hydroxyalkyl starch, it is preferably carried out using an alkaline iodine solution as described, e.g., in DE 196 28 705 A1 the respective contents of which (example A, column 9, lines 6 to 24) is incorporated herein by reference.

According to one embodiment the hydroxyalkyl starch selectively oxidized at its reducing end is reacted with an at least bifunctional compound comprising a functional group Z which is reacted with the oxidized reducing end and a functional group W which is further reacted with a compound which comprises a functional group V being reacted with W and which comprises an alpha-X beta-amino group.

Therefore, also a method as described above, is described said method comprising oxidizing hydroxyalkyl starch at its reducing end, reacting the oxidized reducing end with a functional group Z of a compound comprising, in addition to Z, a further functional group W, to give a first hydroxyalkyl starch derivative, and reacting the functional group W of the first hydroxyalkyl starch derivative with a functional group V of a compound comprising, in addition to V, an alpha-X beta-amino group, to give the alpha-X beta-amino functionalized hydroxyalkyl starch derivative.

According to one described embodiment, both Z and W comprise the chemical structure -NH-.

According to a described embodiment, the functional groups Z and W are a group having the structure R'-NH- where R' is hydrogen or a alkyl, cycloalkyl, aryl, aralkyl, arylcycloalkyl, alkaryl or cycloalkylaryl residue where the cycloalkyl, aryl, aralkyl, arylcycloalkyl, alkaryl or cycloalkylaryl residue may be linked directly to the NH group or, according to another embodiment, may be linked by an oxygen bridge to the NH group. The alkyl, cycloalkyl, aryl, aralkyl, arylcycloalkyl, alkaryl, or cycloalkylaryl residues may be suitably substituted. As preferred substituents, halogenes such as F, Cl or Br may be mentioned. Especially preferred residues R' are hydrogen, alkyl and alkoxy groups, and even more preferred are hydrogen and unsubstituted alkyl and alkoxy groups.

Among the alkyl and alkoxy groups, groups with 1, 2, 3,4, 5, or 6 C atoms are preferred. More preferred are methyl, ethyl, propyl, isopropyl, methoxy, ethoxy, propoxy, and isopropoxy groups. Especially preferred are methyl, ethyl, methoxy, ethoxy, and particular preference is given to methyl or methoxy.

According to another described embodiment, the functional groups Z and W have the structure R'-NH-R"- where R" preferably comprises the structure unit -NH- and/or the structure unit -(C=G)- where G is O or S, and/or the structure unit -SO₂-. According to more preferred embodiments, the functional group R" is selected from the group consisting of 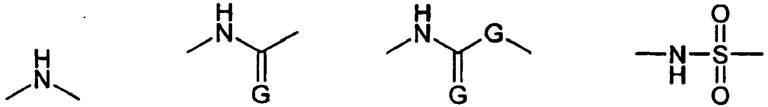 and 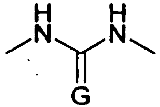 where, if G is present twice, it is independently O or S.

Therefore, also a method and a conjugate as mentioned above are descibed wherein the functional groups Z and W are independently selected from the group consisting of

H₂N-

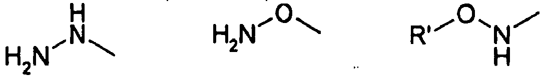

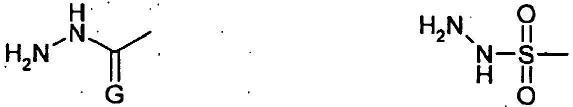

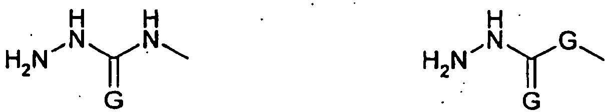

wherein G is O or S and, if present twice, independently O or S, and R' is methyl.

Specific examples for these compounds comprising Z and W are 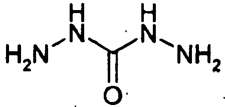 or 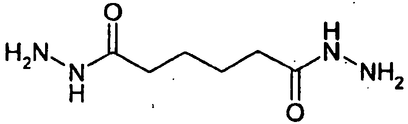 or

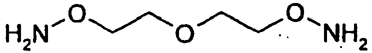

According to a further described embodiment, both functional groups Z and W are an amino group -NH₂.

Therefore, also a method as described above is described, wherein the compound comprising Z and W is a diamino functionalized compound.

In case both Z and W are an amino group -NH₂, Z and W may be separated by any suitable spacer. Among others, the spacer may be an optionally substituted, linear, branched and/or cyclic hydrocarbon residue. Generally, the hydrocarbon residue has from 1 to 60, preferably from 1 to 40, more preferably from 1 to 20, more preferably from 2 to 10, more preferably from 2 to 6 and especially preferably from 2 to 4 carbon atoms. If heteroatoms are present, the separating group comprises generally from 1 to 20, preferably from 1 to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group. According to an even more preferred embodiment, the hydrocarbon residue is an alkyl chain of from 1 to 20, preferably from 2 to 10, more preferably from 2 to 6, and especially preferably from 2 to 4 carbon atoms.

Therefore, also a method and a conjugate as described above are described wherein the hydroxyalkyl starch, preferably at its oxidized reducing end, is reacted with 1,4-diaminobutane, 1,3-diaminopropane or 1,2-diaminoethane to give an amino functionalized hydroxyalkyl starch derivative.

Thus, according to a first alternative, the functional group Z being an amino group NH₂ is reacted with the oxidized reducing end of the hydroxyalkyl starch resulting in an amido group linking the hydroxyalkyl starch and the compound comprising Z and W.

According to a second alternative, the functional group Z being an amino group NH₂ is reacted with the non-oxidized reducing end of the hydroxyalkyl starch via reductive amination resulting in an imino group which is subsequently preferably hydrogenated to give a amino group, the imino group and the amino group, respectively, linking the hydroxyalkyl starch and the compound comprising Z and the amino group W. In this case it is preferred that the compound comprising Z and W is a primary amine which contains - as functional group - only one amino group. In this specific case, although the compound contains only one functional group, it is regarded as bifunctional compound comprising Z an W wherein Z is the amino group contained in the compound subjected to the reductive animation with the reducing end of the polymer, and wherein W is the secondary amino group resulting from the reductive amination and subsequent hydrogenation.

According to a third alternative, the non-oxidized reducing end of the polymer is reacted with ammonia via reductive amination resulting in a terminal imino group of the polymer which is subsequently preferably hydrogenated to give a terminal amino group of the polymer and thus a terminal primary amino group. In this specific case, ammonia is regarded as bifunctional compound comprising Z and W wherein Z is NH₂ comprised in the ammonia employed, and wherein W is the primary amino group resulting from reductive amination and subsequent hydrogenation.

The preferred amino functionalized hydroxyalkyl starch derivative is further reacted with a compound which comprises a functional group V being reacted with W and which comprises an alpha-X beta-amino group.

According to a preferred described embodiment, the compound comprising a functional group V being reacted with W and an alpha-X beta-amino group is a cysteine or histidine derivative.

According to a further preferred embodiment, the cysteine derivative or histidine derivative is pre-activated by a suitable activating agent. Suitable activating agents are, among others, carbodiimides such as diisopropyl carbodiimde (DIC), dicyclohexyl carbodiimides (DCC), 1-ethyl-3-(3-dimethylaminopropyl) carbodiimide (EDC), with diisopropyl carbodiimde (DIC) being especially preferred.

The SH group and the amino group of the cysteine derivate should be suitably protected when the cysteine derivative is reacted with the amino group of the hydroxyalkyl starch derivative. As to the respective protective groups, all suitable compounds known in the art may be employed. Accordingly, if necessary, the group 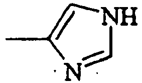 should be suitably protected.

As protective group for the SH group, Acetamidomethyl- (Acm), tert-Butylthio- (StBu),4-Methoxybenzyl- (4-MeOBzl), 4-Methoxyltrityl- (Mmt), Trityl (Trt)- and 4-Methyltrityl-(Mtt) are preferred, StBu being especially preferred.

As protective group for the amino group, tert-Butyloxycarbonyl- (Boc), Fluorenyloxycarbonyl- (Fmoc), 4-Methoxyltrityl- (Mmt), Trityl (Trt)- and 4-Methyltrityl-(Mtt) are preferred Fmoc being especially preferred.

As protective group for 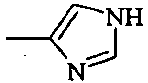 group, tert-Butyloxycarbonyl- (Boc), Benzyloxymethyl (Bom), Dinitrophenyl- (Dnp), 4-Methyltrityl- (Mtt), Tosyl- (Tos) and Trityl (Trt)- are preferred, 4-Methyltrityl- (Mtt) and Trityl (Trt) being especially preferred.

Therefore, also a method as described above is decribed, wherein the compound comprising V and the alpha-X beta-amino is cysteine or histidine or a derivative thereof, V being the carboxy group.

Therefore, the also an alpha-X beta-amino functionalized hydroxyalkyl starch derivative according to formula (VIc) is described 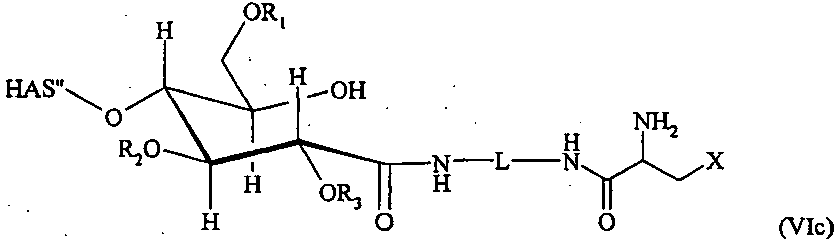 wherein L is an optionally suitably substituted, linear, cyclic and/or branched alkyl, aryl, heteroaryl, aralkyl, for heteroaralkyl residue with 2 to 10 carbon atoms in the respective alkyl residue,
wherein HAS" refers to the HAS molecule without the terminal saccharide unit at the reducing end, and
wherein R₁, R₂ and R₃ are independently hydrogen or a hydroxyalkyl group, preferably a hydroxyethyl group.

According to another embodiment, also method as described above, wherein hydroxyalkyl starch is selectively oxidized at its reducing end, preferably according the method as described above, and the resulting hydroxyalkyl starch is reacted with a functional group Z of a compound comprising, in addition to Z, an alpha-X beta-amino group, is described.

Therefore, also a method as described above, said method comprising oxidizing hydroxyalkyl starch at its reducing end and reacting the oxidized reducing end with a functional group Z of a compound comprising, in addition to Z, an alpha-X beta-amino group, is described.

According to one described embodiment, Z comprises the chemical structure -NH-.

According to one described embodiment, the functional group Z is a group having the structure R'-NH- where R' is hydrogen or a alkyl, cycloalkyl, aryl, aralkyl, arylcycloalkyl, alkaryl or cycloalkylaryl residue where the cycloalkyl, aryl, aralkyl, arylcycloalkyl, alkaryl or cycloalkylaryl residue may be linked directly to the NH group or, according to another embodiment, may be linked by an oxygen bridge to the NH group. The alkyl, cycloalkyl, aryl, aralkyl, arylcycloalkyl, alkaryl, or cycloalkylaryl residues may be suitably substituted. As preferred substituents, halogenes such as F, Cl or Br may be mentioned. Especially preferred residues R' are hydrogen, alkyl and alkoxy groups, and even more preferred are hydrogen and unsubstituted alkyl and alkoxy groups.

Among the alkyl and alkoxy groups, groups with 1, 2, 3, 4, 5, or 6 C atoms are preferred. More preferred are methyl, ethyl, propyl, isopropyl, methoxy, ethoxy, propoxy, and isopropoxy groups. Especially preferred are methyl, ethyl, methoxy, ethoxy, and particular preference is given to methyl or methoxy.

According to another described embodiment, the functional group Z has the structure R'-NH-R"- where R" preferably comprises the structure unit -NH- and/or the structure unit -(C=G)- where G is O or S, and/or the structure unit -SO₂-. According to more preferred embodiments, the functional group R" is selected from the group consisting of 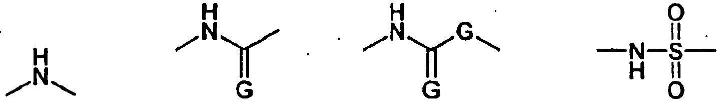 and 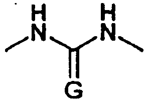 where, if G is present twice, it is independently O or S.

According to a particularly preferred described embodiment the functional group Z is an amino group -NH₂.

The amino group Z and the alpha-thiol beta-amino group may be separated by any suitable spacer. Among others, the spacer may be an optionally substituted, linear, branched and/or cyclic hydrocarbon residue. Generally, the hydrocarbon residue has from 1 to 60, preferably from 1 to 40, more preferably from 1 to 20, more preferably from 1 to 10, more preferably from 1 to 6 and especially preferably from 1 to 4 carbon atoms. If heteroatoms are present, the separating group comprises generally from 1 to 20, preferably from 1 to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group. According to an even more preferred embodiment, the hydrocarbon residue is an alkyl chain of from 1 to 4 such as a methylene, ethylene, propylene, or butylene residue. Particularly preferred is the methylene residue.

Therefore, also a method as described above is described, wherein the compound comprising Z and the alpha-X beta-amino group is 1,3-diamino-2-thiol propane or 2,3-diamino-1-thiol propane, wherein the thiol group may be replaced by the group

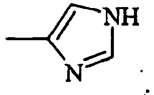

Accordingly, also described is an alpha-thiol beta-amino functionalized hydroxyalkyl starch derivative according to formula (VIa) 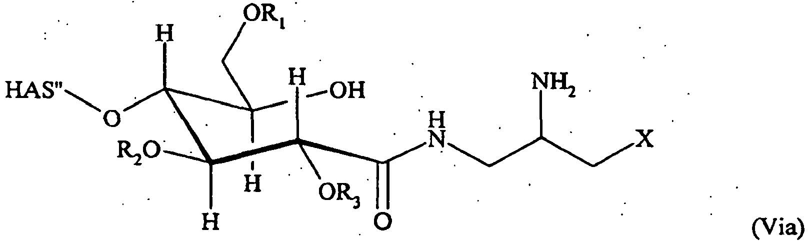 or according to formula (VIb) 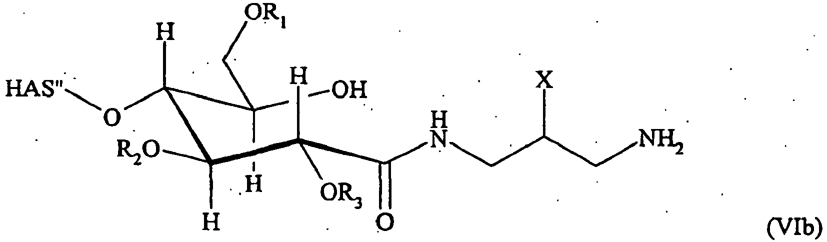 wherein HAS" refers to the HAS molecule without the terminal saccharide unit at the reducing end, and
wherein R₁, R₂ and R₃ are independently hydrogen or a hydroxyalkyl group, preferably a hydroxyethyl group.

The alpha-X beta-amino functionalized hydroxyalkyl starch derivative is then reacted with the thioester functionalized active substance.

According to the native chemical ligation reaction described, the intermediate product of the reaction of the thioester functionalized active substance and the alpha-X beta-amino functionalized hydroxyalkyl starch, is a thioester which is irreversibly converted by intramolecular trans-acetylation to the described conjugate comprising the active substance linked via an amide linkage to the hydroxyalkyl starch derivative.

The reaction of the thioester functionalized compound, i.e. the thioester functionalized active substance or the thioester functionalized hydroxyalkyl starch or derivative thereof, with the alpha-X beta-amino functionalized compound. i.e. the alpha-X beta-amino functionalized hydroxyalkyl starch or derivative thereof or the alpha-X beta-amino functionalized active substance; may be carried out in any suitable solvent or mixture of at least two solvents and at a suitable pH and at a suitable reaction temperature.

The reaction temperature is preferably in the range of from 0 to 40 °C, more preferably or from 10 to 30 °C such as about 20 to 25 °C, like 20 °C, 21 °C, 22 °C, 23 °C, 24 °C, or 25. °C.

The pH the reaction is carried out at is preferably in the range of from 5 to 9, preferably of from 6 to 8 and more preferably of from 6.5 to 7.5.

As buffer media, acetate buffers such as sodium acetate buffer, e.g. having a concentration of about 0.1 M, bicarbonate buffers such as ammonium bicarbonate buffer, e.g. having a concentration of about 0.5 M, and/or phosphate buffers such as sodium phosphate buffer, e.g. having a concentration of about 0.1 M may be mentioned.

The reaction time is preferably in the range of up to 48 h, more preferably of from 1 to 48 h, more preferably of from 8 to 32 h and more preferably of from 20 to 24 h.

The reaction may be carried out in any suitable solvent. A preferred solvent is water. To the solution of the thioester functionalized compound and/or the alpha-X beta-amino functionalized compound, at least one suitbable catalyst and/or at least one adjuvant may be added.

Preferred catalysts are, among others, thiophenol in a concentration of about, e.g., from 2 to 6 % v/v, preferably of from 3 to 5 % v/v, or benzyl mercaptane, or MESNA in a concentration of about, e.g., from 0.4 to 0.6 M such as about 0.5 M, and/or tris(carboxyethyl)phosphine or a mixture of two or more thereof.

Preferred adjuvants are, among others, urea in a concentration of about 1 to 8 M, preferably of from 1 to 7 M, more preferably of from 1 to 6 M, more preferably of from 1 to 5 M, more preferably of from 1 to 4 M, more preferably of from 1 to 3 M such as about 2 M, or guanidinium hydrochloride in a concentration of about 4 to 8 M, preferably of from 5 to 7 M such as about 6 M, a suitable organic solvent such as DMF or acetonitrile in a concentration of about 20 to 40 % v/v, preferably about 25 to 35 % v/v; or 3-[(3-cholamidopropyl)dimethylammonio]-1-propane sulfonate (CHAPS) in a concentration of about from 2 to 8 % v/v, preferably of from 3 to 7 % v/v, more preferably of from 4 to 6 % v/v such as about 5 % v/v, or sodium chloride in a concentration of about from 0.1 to 0.5 M, preferably of from 0.2 to 0.4 M such as about 0.3 M.

The concentration of the thioester functionalized compound and the alpha-X beta-amino functionalized compound, respectively, in the reaction solution is preferably in the range of from 0.001 to 0.5 M, more preferably of from 0.02 to 0.4 M, more preferably of from 0.05 M to 0.3 M, more preferably of from 0.1 to 0.2 M.

According to one embodiment described herein, the active substance is a small molecule drug comprising a carboxy group. In this case, the carboxy group of the active substance is suitably converted in a thioester group -S-R', e.g. by
- suitably converting the oxidized reducing end of the hydroxyalkyl starch to an acid chloride and reacting the acid chloride with a mercaptane R'-SH via alkylthio-dehalogenation (J. March, Advanced Organic Chemistry, 4th edition, John Wiley and Sons, New York (1992) 409, or other methods cited therein (paragraph 0-37), or
- converting the oxidized reducing end of the hydroxyalkyl starch to an acid chloride by reaction with a thallium(I) salt of a thiolate (Spessard, G., et al., Organic Synthesis Collection, vol. 7, 87), or
- by reacting the carboxylic acid-terminated hydroxyalkyl starch by reaction of the acid with a dialkyl or diphenyl phosphorochloridate to form an anhydride which can then be converted to the corresponding thioester (Masamune, S., et al., Can. J. Chem. 53 (1975) 3693), or
- by converting the oxidized reducing end of the hydroxyalkyl starch to an imidazolide of a carboxylic acid, prepared, e.g., by reaction of the corresponding carboxylic acid with, e.g., N,N-carbonyldiimidazole) with a comparatively acidic thiol (Masamune, S., et al., J. Am. Chem. Soc. 98 (1976) 7874), or
- by converting the oxidized reducing end of the hydroxyalkyl starch using a disulfide and triphenylphosphine to the corresponding thioester (Mukaiyama, T., et al., Bull. Chem. Soc. Jpn. 43 (1970) 1271), or
- reacting the oxidized reducing end of the hydroxyalkyl with aryl thioisocyanates (Grieco, P., et al., Tetrahedron Lett. 43 (1979) 1283), or
- reacting the oxidized reducing end of the hydroxyalkyl with thiopyridyl chloroformate (Corey, E.J., et al., Tetrahedron Lett. (1979), 2875), or
- reacting the oxidized reducing end of the hydroxyalkyl with 2-fluoro-N-methylpyridinium tosylate (Watanabe, Y., et al., Chem. Lett (1976) 741), or
- reacting the oxidized reducing end of the hydroxyalkyl with 1-hydroxybenzotriazole (Pelter, A., et al., J. Chem Soc., Perkin trans I (1977) 1672).

It is preferred that the carboxy group of the active substance be converted to a thioester group -S-R' by
- suitably converting the oxidized reducing end of the hydroxyalkyl starch to an acid chloride and reacting the acid chloride with a mercaptane R'-SH via alkylthio-dehalogenation (J. March, Advanced Organic Chemistry, 4th edition, John Wiley and Sons, New York (1992) 409, or other methods cited therein (paragraph 0-37),
or by
- reacting the oxidized reducing end of the hydroxyalkyl starch with a carbodiimide such as such as diisopropyl carbodiimde (DIC), dicyclohexyl carbodiimides (DCC), 1-ethyl-3-(3-dimethylaminopropyl) carbodiimide (EDC), on a solid support immobilized EDC and a thiol. (C.E-Lin et al. Tetrahedron Lett. 43 (2002) 4531-34; M. Adamczyk, Tetrahedron Lett. 37 (1996) 4305-8, J. Hovinen, Nucleosides Nucleotides 18 (1999) 1263-4).

According to another embodiment described herein, the active substance is an artificially produced peptide. According to one preferred alternative of the present invention, the thioester functionalized peptide is prepared using a suitable synthesis resin allowing for thioester functionalized peptides.

Thioester functionalized peptides can be synthesized using Boc chemistry as well as Fmoc chemistry. Tam et al. describe synthesis of thioester functionalized peptides using Boc chemistry on the acid-labile Trt-mercapto-propionyl-MBHA resin (Tam., J.P., et al., Proc. Natl. Acad. Sci. USA 92 (1995) 12485). According to an especially preferred embodiment, the Trt group is removed with TFA/DCM. Following washing and neutralization with DIPEA, the resin can be loaded with the C-terminal residue of the peptide using DIPCDI/HOBt activation. It is preferred that unreacted thiol sites are then capped with AcCl/DIPEA in DCM. After chain extension using standard Boc protocols, HF cleavage provides the side chain deprotected thioester functionalized peptide.

Li et al. describe the synthesis of thioester functionalized peptides using Fmoc chemistry (Li, X. et al., (1998) Tetrahedron Letters 39(47):8669-8672). In a preferred embodiment, a mixture of 1-methylpyrrolidine, hexamethyleneimine and HOBt is used as the deblocking agent for removal of Fmoc, preferably in a mixture of NMP and DMSO, more preferably wherein the molar ratio of NMP and DMSO is from 1:10 to 10:1, even more preferably from 1:5 to 5:1, most preferably from 1:2 to 2:1. The resins to be used can be, for example, the resins cited in Li et al. or cited in Goldstein and Gelb (2000) Tetrahedron Letters 41(16):2797-2800.

Another possibility for producing thioester functionalized peptides is also based on Fmoc chemistry and makes use of a sulfamylbutyryl safety-catch linked employing thiolytic cleavage, for example from the 4-Sulfamylbutyryl NovaSyn TG resin (Shin, Y., et al., J. Am. Chem. Soc. 121 (1999), 11684; Ingenito, R., et al., J. Am. Chem Soc. 121 (1999) 11369). Synthesis resins modified with the mentioned linkers are commercially available for both, the Boc chemistry and the Fmoc chemistry (e.g. from Novabiochem, Merck Biosciences GmbH, Schwalbach, Germany).

Therefore, also described is a method for producing a thioester functionalized peptide, said method comprising producing the peptide by chemical solid phase peptide synthesis on a synthesis resin allowing for the C-terminal thioester formation in the final cleaving step form the solid support after side chain deprotection.

According to a further embodiment of the present invention, the active substance is a protein.

The protein can be produced by chemical synthetic procedures or can be of any human or another mammalian source and can be obtained by purification from naturally occurring sources like human or animal. In this case, it is possible the produce a derivative of the protein by reacting at least one functional group of the protein with a suitable compound which comprises at least one functional group which is reacted with a functional group of the protein and at least one other functional group which is a thioester group or a group G which can be chemically modified to give a thioester group. Such a chemical modification may be a reaction of this functional group G with a further compound which comprises a functional group which is reacted with G and another functional group which is a thioester group -S-R'.

As functional group of the protein, a hydroxyl group, an amino group, a thiol group, a carboxy group, a keto group, an aldehyde group or a hemiacetale group may be mentioned. The keto group or the aldehyde group or the hemiacetal group may be produced by chemically or enzymatically oxidizing a saccharide unit of the protein such as a saccharide unit of a carbohydrate side chain of a glycosylated protein.

Depending on the chemical nature of the functional group of the protein, the functional group of the suitable compound which is reacted with the functional group of the protein is, e.g.:
- C-C-double bonds or C-C-triple bonds or aromatic C-C-bonds;
- the thio group or the hydroxy groups;
- alkyl sulfonic acid hydrazide, aryl sulfonic acid hydrazide;
- 1,2-dioles;
- 1,2 amino-thioalcohols;
- azides;
- 1,2-aminoalcohols;
- the amino group -NH₂ or derivatives of the amino groups comprising the structure unit -NH- such as aminoalkyl groups, aminoaryl group, aminoaralkyl groups, or alkarlyaminogroups;
- the hydroxylamino group -O-NH₂, or derivatives of the hydroxylamino group comprising the structure unit -O-NH-, such as hydroxylalkylamino groups, hydroxylarylamino groups, hydroxylaralkylamino groups, or hydroxalalkarylamino groups;
- alkoxyamino groups, aryloxyamino groups, aralkyloxyamino groups, or alkaryloxyamino groups, each comprising the structure unit -NH-O-;
- residues having a carbonyl group, -Q-C(=G)-M, wherein G is O or S, and M is, for example,
   -- -OH or -SH;
   -- an alkoxy group, an aryloxy group, an aralkyloxy group, or an alkaryloxy group;
   -- an alkylthio group, an arylthio group, an aralkylthio group, or an alkarylthio group;
   -- an alkylcarbonyloxy group, an arylcarbonyloxy group, an aralkylcarbonyloxy group, an alkarylcarbonyloxy group;
   -- activated esters such as esters of hydroxylamines having imid structure such as N-hydroxysuccinimide or having a structure unit O-N where N is part of a heteroaryl compound or, with G = O and Q absent, such as aryloxy compounds with a substituted aryl residue such as pentafluorophenyl, paranitrophenyl or trichlorophenyl;
   wherein Q is absent or NH or a heteroatom such as S or O;
- -NH-NH₂, or -NH-NH-;
- -NO₂;
- the nitril group;
- carbonyl groups such as the aldehyde group or the keto group;
- the carboxy group;
- the -N=C=O group or the -N=C=S group;
- vinyl halide groups such as the vinyl iodide or the vinyl bromide group or triflate;
- -C≡C-H;
- -(C=NH₂Cl)-OAlkyl
- groups -(C=O)-CH₂-Hal wherein Hal is Cl, Br, or I;
- -CH=CH-SO₂-;
- a disulfide group comprising the structure -S-S-;
- the group 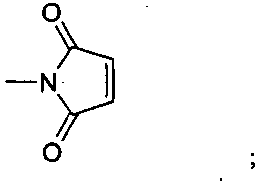
- the group

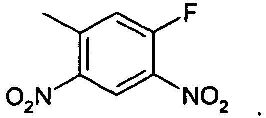

The functional group of the suitable compound and the thioester group or the group G may be separated by any suitable spacer. Among others, the spacer may be an optionally substituted, linear, branched and/or cyclic hydrocarbon residue. Generally, the hydrocarbon residue has from 1 to 60, preferably from 1 to 40, more preferably from 1 to 20, more preferably from 2 to 10, more preferably from 2 to 6 and especially preferably from 2 to 4 carbon atoms. If heteroatoms are present, the separating group comprises generally from 1 to 20, preferably from I to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group.

In case the group G is chemically modified with a further compound which comprises a functional group which is reacted with G and another functional group which is a thioester group -S-R', the functional group which is reacted with G may be suitably selected from the group of functional groups described above with regard to Q. The thioester group and the group which is reacted with G may be separated by any suitable spacer. Among others, the spacer may be an optionally substituted, linear, branched and/or cyclic hydrocarbon residue. Generally, the hydrocarbon residue has from 1 to 60, preferably from 1 to 40, more preferably from 1 to 20, more preferably from 2 to 10, more preferably from 2 to 6 and especially preferably from 2 to 4 carbon atoms. If heteroatoms are present, the separating group comprises generally from 1 to 20, preferably from 1 to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group.

Preferably, the protein is recombinantly produced. This includes prokaryotic or eukaryotic host expression of exogenous DNA sequences obtained by genomic or cDNA cloning or by DNA synthesis. The recombinant production of a protein is known in the art. In general, this includes the transfection of host cells with an appropriate expression vector, the cultivation of the host cells under conditions which enable the production of the protein and the purification of the protein from the host cells.

According to a still further preferred embodiment, an expression vector is used which leads to a thioester functionalized protein, preferably a C-terminal polypeptide-thioester. In one embodiment, the thioester functionalized protein is bound via a C-terminal thioester bond to an intein polypeptide, preferably to an intein affinity-tag fusion polypeptide. Such thioester functionalized proteins are, for example, obtainable by fusing the protein of interest in frame with a, preferably mutant, intein-CBD polypeptide, for example in the context of the vectors of the commercially available IMPACT (TM) - CN system, like pCYB or pTYB (New England Biolabs, Frankfurt/Main, Germany). The thioester functionalized protein bound via a thioester bond to the Intein (TFPI) preferably comprises an affinity tag fused to the C-terminus of the intein, more preferably the affinity tag can be a chitin-binding domain (CBD), a polyhistidine-tag, a Strep-tag or GST. In one embodiment, the TFPI is further bound via an affinity tag to a corresponding affinity resin, for example bound to a chitin-bead via a CBD fused to the intein (see, for example, Muir et al. (1998) Proc. Natl. Acad. Sci. USA 95:6705-6710). In a preferred embodiment, the TFPI is purified on the affinity resin under conditions leaving the thioester linkage intact (see Muir et al., above). The TFPI bound to the affinity resin can then be reacted with the alpha-X beta-amino functionalized HES, preferably to alpha-X beta-amino functionalized HES such as alpha-X beta-amino functionalized HES with a mean molecular weight of about 10 kD.

It is preferred that a thioester functionalized protein bound via a thioester bond to an alkyl-, aryl- or aralkyl-residue (TFPA) be generated, e.g. by reacting the TFPI with an alkylthiol, arylthiol or aralkylthiol. In a preferred embodiment, the alkylthiol is not a dithiol and is selected from methyl-, ethyl-, propyl- or butylthiol; ethylthiol is particularly preferred. The alkylthiol may also be a mercaptoalkylcarbonic acid or a mercaptoalkylsulfonic acid. 2-mercaptoethansulfonic acid is particularly preferred. In another embodiment, the arylthiol is not a dithiol and can be, for example, thiophenol, 1-thio-2-nitrophenol, 2-thio-benzoic acid, 2-thiopyridine, 4-thio-2-pyridine carboxylic acid or 4-thio-2-nitropyridin. The TFPA can be isolated, as described, e.g., in US 2002/0151006A1 in Example 3 for an Abl-SH3 ethyl-thioester, and can either be used directly for the reaction with the alpha-X beta-amino functionalized HES or can even be purified further and then used for reaction or can even be stored and then be used for the reaction later. A further example of a TFPA is shown in Iakovenko, A., et al., FEBS Letters 468 (2000) 155-158, where a thioester functionalized Rab7ΔC6 was generated in section 2.2. (a thioester with 2-mercaptoethansulfonic acid), purified and stored before use for a final coupling step.

Therefore, also described is a method as described above, wherein the active substance is a protein which was produced using an expression vector leading to a thioester functionalized protein.

According to another aspect, the present invention also relates to a conjugate obtainable by a method as described above.

It is envisaged that the method according to the present invention is a chemoselective method, i.e. depending on the alternatives described above, coupling of hydroxyalkyl starch or a derivative thereof, preferably hydroxyethyl starch or a derivative thereof, takes place at specific sites of the active substance such as selectively at the N-terminus of the active substance in case the active substance has a N-terminal cysteine residue, as described above, or selectively at the C-terminus of the active substance in case the active substance has a C-terminal thioester group as described above, wherein the active substance is reacted with a thioester functionalized hydroxyalkyl starch derivative and with an alpha-thiol beta-amino functionalized hydroxyalkyl starch derivative, respectively.

The term "selectively at a terminus" as used in the context of the present invention relates to a process according to which statistically more than 50 %, preferably at least 55 %, more preferably at least 60 %, more preferably at least 65 %, more preferably at least 70 %, more preferably at least 75 %, more preferably at least 80 %, more preferably at least 85 %, more preferably at least 90 %, and still more preferably at least 95 % such as 95 %, 96 %, 97 %, 98 %, or 99 % of active substance molecules are exclusively reacted via the respective terminus.

The present invention also comprises an embodiment in which the thiol group of the alpha-thiol beta-amino group is replaced by a group according to the formula

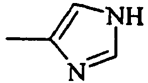

Therefore, instead of a cystein, a histidin may be employed, preferably an N-terminal cysteine or histidine.

In the methods for preparing a conjugate of the invention the conversion rate in the above described methods may be at least 50%, more preferred at least 70%, even more preferred at least 80% and in particular 95% or even more, such as at least 98% or 99%.

According to yet another aspect, the present invention also relates to a conjugate as described above or a conjugate, obtainable by a method as described above, for use in a method for the treatment of the human or animal body.

The conjugates according to the invention may be at least 50% pure, even more preferred at least 70% pure, even more preferred at least 90%, in particular at least 95% or at least 99% pure. In a most preferred embodiment, the conjugates may be 100% pure, i.e. there are no other by-products present.

Therefore, according to another aspect, the present invention also relates to a composition which may comprise the conjugate(s) of the invention, wherein the amount of the conjugate(s) may be at least 50 wt-%, even more preferred at least 70 wt-%, even more preferred at least 90 wt-%, in particular at least 95 wt.% or at least 99 wt.%. In a most preferred embodiment, the composition may consist of the conjugate(s), i.e. the amount of the conjugate(s) is 100 wt.-%.

Accordingly, the present invention also relates to a pharmaceutical composition comprising a conjugate as described above or a conjugate, obtainable by a method as described above.

Moreover, the present invention also relates to a pharmaceutical composition comprising a conjugate as described above or a conjugate, obtainable by a method as described above, said pharmaceutical composition further comprising at least one pharmaceutically acceptable diluent, adjuvant, or carrier.

### Short description of the Figures

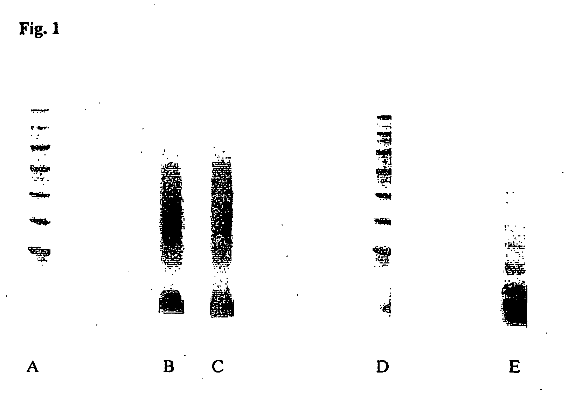

- **Fig. 1**: shows an analysis of the crude peptide-thioester Cys-HES conjugates according to example 2.1 by gel electrophoresis.
For gel electrophoresis a XCell Sure Lock Mini Cell (Invitrogen GmbH, Karlsruhe, D) and a Consort E143 power supply (CONSORTnv, Turnhout, B) were employed. A 12% Bis-Tris gel together with a MES SDS running buffer at reducing conditions (both Invitrogen GmbH, Karlsruhe, D) were used according to the manufacturer's instruction:
Lane A: Roti®-Mark STANDARD (Carl Roth GmbH + Co. KG, Karlsruhe, D) Molecular weight marker from top to bottom: 200 KD, 119 KD, 66 KD, 43 KD, 29 KD, 20 KD, 14.3 KD;
Lane B: Conjugation of Peptide-Thioester A to H-Cys(S-tBu)-HES10/0.4. employing thiophenol as catalyst;
Lane C: Conjugation of Peptide-Thioester A to H-Cys(S-tBu)-HES10/0.4 employing benzyl mercaptan as catalyst;
Lane D: Roti®-Mark STANDARD;
Lane E: Conjugation of Peptide-Thioester A to H-Cys(S-tBu)-HES10/0.4 without catalyst.

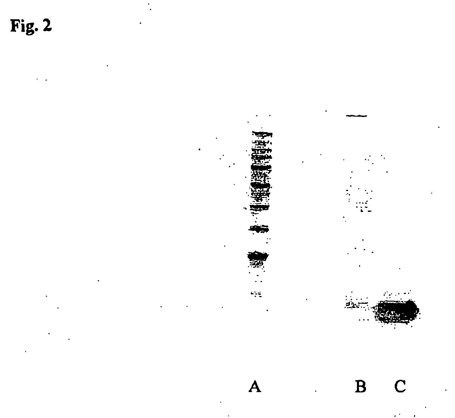

- **Fig. 2**: shows an analysis of the crude Cys-peptide thioester-HES conjugates according to example 2.2 by gel electrophoresis.
For gel electrophoresis a XCell Sure Lock Mini Cell (Invitrogen GmbH, Karlsruhe, D) and a Consort E143 power supply (CONSORTnv, Turnhout, B) were employed. A 12% Bis-Tris gel together with a MES SDS running buffer at reducing conditions (both Invitrogen GmbH, Karlsruhe, D) were used according to the manufacturer's instruction:
Lane A: Roti®-Mark STANDARD (Carl Roth GmbH + Co. KG, Karlsruhe, D) Molecular weight marker from top to bottom: 200 KD, 119 KD, 66 KD, 43 KD, 29 KD, 20 KD, 14.3 KD;
Lane B: Conjugation of Thioester-HES10/0.4 to GM-CSF Inhibitor Peptide (54-78) employing thiophenol as catalyst;
Lane C: GM-CSF Inhibitor Peptide (54-78).

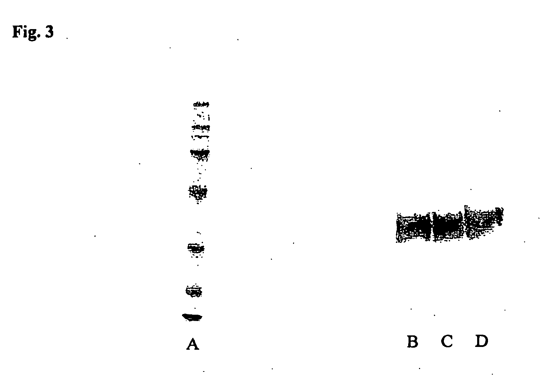

- **Fig.3**: shows an Analysis of the crude EPO thioester-HES conjugates according to example 2.3 by gel electrophoresis.
For gel electrophoresis a XCell Sure Lock Mini Cell (Invitrogen GmbH, Karlsruhe, D) and a Consort E 143 power supply (CONSORTnv, Turnhout, B) were employed. A 10% Bis-Tris gel together with a MOPS SDS running buffer, at reducing conditions (both Invitrogen GmbH, Karlsruhe, D) were used according to the manufactures instruction.
Lane A: Roti®-Mark STANDARD (Carl Roth GmbH + Co. KG, Karlsruhe, D) Molecular weight marker from top to bottom: 200 KD, 119 KD, 88 KD, 43 KD, 29 KD, 20 KD, 14.3 KD;
Lane B: Conjugation of 6.9 equiv. thioester-HES to EPO employing thiophenol as catalyst;
Lane C: Conjugation of 34.5 equiv. thioester-HES to EPO employing thiophenol as catalyst;
Lane D: EPO without thioester-HES, employing thiophenol as catalyst.

### Examples

### Example 1 Synthesis of functionalized HES:

### Example 1.1 Synthesis of amino-HES from oxidized HES

5.122 g of HES (MW = 14,500 D, DS = 0.41, Supramol Parenteral Colloids GmbH, Rosbach-Rodheim, D), selectively oxidized at its reducing end according to DE 196 28 705 A1, were heated at 80 °C in vaccuo for 15.5 h and dissolved under nitrogen in 25 mL dry DMSO (Fluka, Sigma-Aldrich Chemie GmbH, Taufkirchen, D). To the resulting solution, 51.22 mmol 1,4-diaminobutane (Fluka, Sigma-Aldrich Chemie GmbH Taufkirchen, D) were added. After incubation for 17 h at 40 °C, the reaction mixture was added to 150 mL of an ice-cold 1:1 mixture of ethanol (DAB, Sonnenberg, Braunschweig, D) and acetone (Carl Roth GmbH + Co: KG, Karlsruhe, D) (v/v), and incubated at -20 °C for 1 h. The precipitated product was collected by centrifugation at 4 °C, washed with 40 ml of the same mixture, re-dissolved in 80 mL water, dialysed for 40 h against water (SnakeSkin dialysis tubing, 3.5 kD cut off, Perbio Sciences Deutschland GmbH, Bonn, D), and lyophilized. The product was isolated in 67 % yield.

### Example 1.2 (not according to the invention) Syntheses of H-Cys(S-tBu)-HES from amino-HES of example 1.1

86.3 mg Fmoc-Cys(S-tBu)-OH (Fluka, Sigma-Aldrich Chemie GmbH, Taufkirchen, D) and 45.9 mg 1-hydroxy-1H-benzotriazole (Aldrich, Sigma-Aldrich Chemie GmbH, Taufkirchen, D) were dissolved in 2 mL N,N-dimethylformamide (Peptide synthesis grade, Biosolve, Valkenswaard, NL), and 40.7 µL N,N'-diisopropylcarbodiimide (Fluka, Sigma-Aldrich Chemie GmbH, Taufkirchen, D) were added.

After incubation at 21 °C for 30 min, 206 mg of amino-HES, synthesized as described in example 1.1, were added. After shaking for 19 h at 22 °C, the reaction mixture was added to 14 mL of ice-cold 2-propanol (Carl Roth GmbH, Karlsruhe, D). The precipitated product was collected by centrifugation at 4 °C, re-suspended in 8 ml 2-propanol and centrifuged as described. The precipitate was dissolved in 20 mL water, 20 ml dichloromethane (Carl Roth GmbH, Karlsruhe, D) were added and the mixture was vortexed. After centrifugation, the upper aqueous layer was isolated, dialysed for 43 h against water (SnakeSkin dialysis tubing, 3.5 kD cut off, Perbio Sciences Deutschland GmbH, Bonn, D) and lyophilized. Fmoc-Cys(S-tBu)-HES was isolated in 67 % yield.

100.7 mg of Fmoc-Cys(S-tBu)-HES was dissolved in 1 mL of 20 % piperidine (Acros Organics, Geel, B) in DMF (v/v). After shaking for 15 min at 22 °C, the reaction mixture was added to 20 mL of *tert*-butyl methyl ether (Acros Organics, Geel, B). The precipitated product was collected by centrifugation at 4 °C, re-suspended in 10 ml *tert*-butyl methyl ether and centrifuged. After drying, the precipitate was dissolved in 10 mL water and dialysed for 31 h against water (SnakeSkin dialysis tubing, 3.5 kD cut off, Perbio Sciences Deutschland GmbH, Bonn, D) and lyophilized. H-Cys(S-tBu)-HES was isolated in 80% yield.

### Example 1.3 Synthesis of Thioester-HES from amino-HES of example 1.1

29.3 mg Pentafluorophenyl S-benzyl thiosuccinate (Link technologies, Bellshill, UK) and 10.1 mg 1-hydroxy-1H-benzotriazole (Aldrich, Sigma-Aldrich Chemie GmbH, Taufkirchen, D) were dissolved in 1.5 mL N,N-dimethylformamide (Peptide synthesis grade, Biosolve, Valkenswaard, NL), and 75 mg amino-HES, synthesised as described in example 1.1, were added. After shaking for 15 h at 22 °C, the reaction mixture was added to 15 mL of *tert*-butyl methyl ether (Acros Organics, Geel, B). The precipitated product was collected by centrifugation at 4 °C, re-suspended in 8 ml *tert*-butyl methyl ether and centrifuged. The precipitate was dried in a stream of nitrogen.

### Examples 2 synthesis of protein-HES conjugates by chemical ligation:

### Example 2.1 (not according to the invention) Synthesis of a Peptide-Thioester A-HES conjugate from H-Cys(S-tBu)-HES and Peptide-Thioester A

To 5 µL of a 10 mg/mL solution of the peptide thioester Peptide-Thioester A (GBF, Braunschweig, D, amino acid sequence:
H-SPFGADTTVCCFNYSVRKLPQNHVKDYFYTSSK-thiopropionic acid ethyl ester; MW = 3,920 g/mol) in a 1 : 1 mixture (v/v) of N,N-dimethylformamide (peptide synthesis grade, Biosolve, Valkenswaard, NL) and 0.1 M sodium phosphate buffer, pH 7.2, 5 µL of a 255 mg/mL solution of H-Cys(S-tBu)-HES, synthesized as described in example 1.2, and 5 µL of a 1.5 M catalyst solution in DMF were added at 22 °C. As catalyst, either thiophenol, (Fig. 1, Lane B) or benzyl mercaptan (Fig. 1, Lane C) were employed. As a reaction control, 5 µL buffer instead of the catalyst solution were added to the mixture of HES-derivative and peptide (Fig. 1, Lane E).

The mixtures were incubated over night at room temperature and analysed by gel electrophoresis (Figure 1).

### Example 2.2 Synthesis of a GM-CSF Inhibitor Peptide-HES conjugate from Thioester-HES and GM-CSF Inhibitor Peptide

To 10 µL of a 2.82 mg/mL solution of the N-terminal Cys-peptide GM-CSF Inhibitor Peptide (54-78) (Bachem AG, Order-No. H-3438, Bubendorf, CH, Amino acid sequence : H-Cys-Leu-Gln-Thr-Arg-Leu-Glu-Leu-Tyr-Lys-Gln-Gly-Leu-Arg-Gly-Ser-Leu-Thr-Lys-Leu-Lys-Gly-Pro-Leu-Thr-OH; MW = 2,816.4 g/mol) in 0.1. M sodium phosphate buffer, pH 7.2, containing 150 mM sodium chloride and 5 mM EDTA, 32 µL of a 156 mg/mL solution of Thioester-HES, synthesized as described in example 1.3, in the same buffer and 1.7 µL of thiophenol (Fig. 2, Lane B) were added at 22 °C.

The mixture was incubated over night at room temperature and analysed by gel electrophoresis (Figure 2).

### Example 2.3 Reaction control - incubation of EPO with thioester-HES

To 19.2 µL of a 0.78 mg/mL solution of EPO (recombinantly produced EPO having the amino acid sequence of human EPO and essentially the same characteristics as the commercially available Erypo® (Ortho Biotech, Jansen-Cilag) or NeoRocormon® (Roche), cf. EP 0 148 605, EP 0 205 56.4, EP 0 411 678) in 10 mM sodium phosphate buffer, pH 7.2, containing 150 mM sodium chloride, 1.7 µL of thiophenol and 5 µL of either a 10 or 50 mg/mL solution of thioester-HES, synthesized as described in example 1.3, in 0.1 M sodium phosphate buffer, pH 7.2, containing 150 mM sodium chloride and 50 mm EDTA (Fig. 3, Lanes B and C) were added at 22 °C. As a reaction control, the same reaction without thioester-HES was performed (Fig. 3, Lane D).

The mixture was incubated over night at room temperature and analysed by gel electrophoresis.

## Claims

1. A method for preparing a conjugate of an active substance and hydroxyalkyl starch, wherein the active substance and the hydroxyalkyl starch are covalently linked by a chemical residue having a structure according to formula (I) 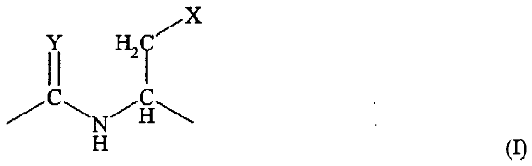 wherein Y is a heteroatom, selected from the group consisting of O and S, said method comprising
reacting a thioester group -(C=Y)-S-R' of a hydroxyalkyl starch derivative comprising said thioester group with an alpha-X beta amino group 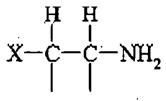 of an active substance derivative comprising said alpha-X beta amino group, said method further comprising
reacting a hydroxyalkyl starch with an at least bifunctional compound comprising a functional group M which is reacted with the hydroxyalkyl starch via the non-oxidized reducing end, and a functional group Q which is a thioester group or a functional group which is further modified to give a thioester group,
to give the hydroxyalkyl starch derivative comprising said thioester group, wherein R' is selected from the group consisting of hydrogen, an optionally suitably substituted, linear, cyclic and/or branched alkyl, aryl, heteroaryl, aralkyl, and heteroaralkyl group, preferably benzyl,
wherein X is selected from the group consisting of SH and 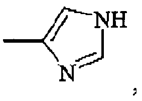 and wherein the group -(C=Y) is derived from the thioester group -(C=Y)-S-R' and the group HN-CH-CH₂-X is derived from the alpha-X beta amino group, and wherein the functional group M is selected from the group consisting of 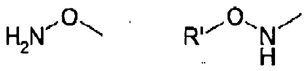 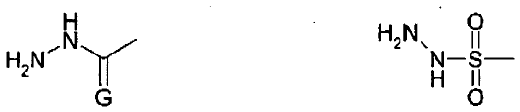 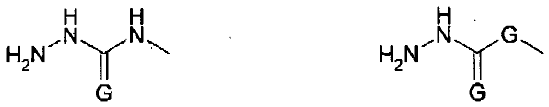 wherein G is O or S and, if present twice, independently O or S, and R' is methyl.

2. The method as claimed in claim 1, wherein the a thioester functionalized hydroxyalkyl starch is reacted with an alpha-X beta-amino group of the active substance wherein the alpha-X beta-amino group is comprised in a cysteine or histidine residue of the active substance.

3. The method as claimed in claim 1 or 2, wherein the functional group Q comprises the chemical structure NH-.

4. The method as claimed in any of claims 1 to 3, wherein the functional group Q is selected from the group consisting of
H₂N- 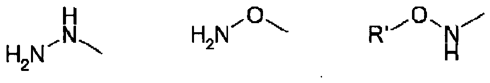 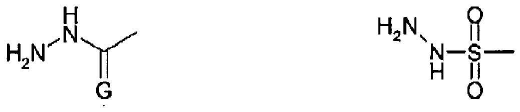 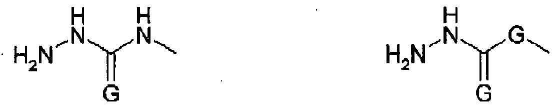
wherein G is O or S and, if present twice, independently O or S, and R' is methyl.

5. The method as claimed in any of claims 1 to 4, wherein the functional group Q is an amino group -NH₂.

6. A conjugate of an active substance and Hydroxyalkyl starch, wherein the active substance and the hydroxyalkyl starch are linked by a chemical moiety, having a structure according to formula (IV) 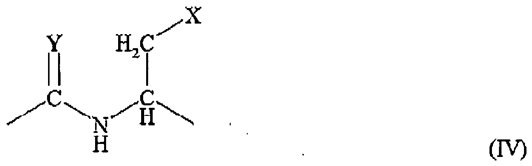 wherein Y is a heteroatom, selected from the group consisting of O and S, and X is
selected from the group consisting of SH and 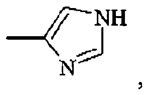 said conjugate having a structure according to formula (II) 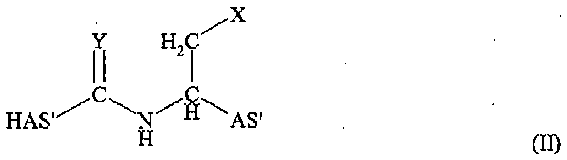 wherein HAS' is the residue of the hydroxyalkyl starch derivative which was linked to the thioester group by reacting the hydroxyalkyl starch with an at least bifunctional compound comprising a functional group M which is reacted with the hydroxyalkyl starch via the non-oxidized reducing end, and a functional group Q which is a thioester group or a functional group which is further modified to give a thioester group, to give the hydroxyalkyl starch derivative comprising said thioester group, and wherein AS' is the residue of the active substance or a derivative thereof which was linked to the alpha-X beta-amino group, and wherein the functional group M is selected from the group consisting of 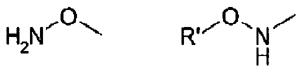 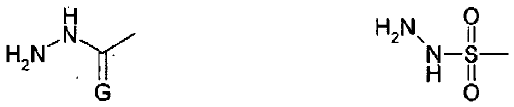 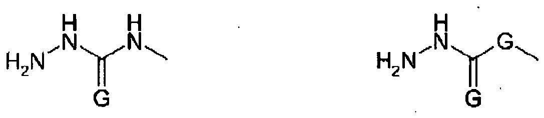 wherein G is O or S and, if present twice, independently O or S, and R' is methyl, and wherein the group -(C=Y) is derived from the thioester group -(C=Y)-S-R' and the group HN-CH-CH₂-X is derived from the alpha-X beta amino group.

7. The conjugate as claimed in claim 6, wherein the active substance is selected from the group consisting of proteins, peptides, and small molecule drugs.

8. The conjugate as claimed in claims 6 to 7, wherein the hydroxyalkyl starch is hydroxyethyl starch having a mean molecular weight of from 1 to 300 kD, preferably from 2 to 200 kD, more preferably from 4 to 130 kD; and a degree of substitution in the range of from 0.1 to 0.8.

9. A conjugate as claimed in any of claims 6 to 8 for use in a method of treatment of the human or animal body.

10. A pharmaceutical composition comprising a conjugate as claimed in any of claims 6 to 8.

11. A pharmaceutical composition as claimed in claim 10, further comprising at least one pharmaceutically acceptable diluent, adjuvant, or carrier.

12. A composition comprising a conjugates of an active substance and hydroxyalkyl starch as claimed in any of claims 6 to 8.

## Patentansprüche

1. Verfahren zur Herstellung eines Konjugats aus einem Wirkstoff und Hydroxyalkylstärke, wobei der Wirkstoff und die Hydroxyalkylstärke über einen chemischen Rest der Struktur gemäß Formel (I) 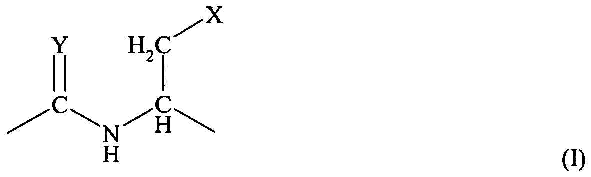 kovalent gebunden sind, wobei Y ein Heteroatom ist, ausgewählt aus der Gruppe bestehend aus O und S, wobei das besagte Verfahren das Umsetzen einer Thioestergruppe -(C=Y)-S-R' eines Hydroxyalkylstärkederivates, enthaltend besagte Thioestergruppe, mit einer alpha-X beta-Aminogruppe 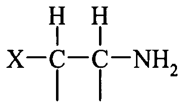 eines Wirkstoffderivates, enthaltend besagte alpha-X beta-Aminogruppe, umfasst, wobei das besagte Verfahren weiterhin das Umsetzen einer Hydroxyalkylstärke mit einer zumindest bifunktionellen Verbindung, enthaltend eine funktionelle Gruppe M, die mit der Hydroxyalkylstärke über das nicht-oxidierte reduzierende Ende umgesetzt wird, und eine funktionelle Gruppe Q, die eine Thioestergruppe oder eine funktionelle Gruppe ist, die weiter modifiziert wird, um eine Thioestergruppe zu erhalten, umfasst, um ein Hydroxyalkylstärkederivat zu erhalten, das besagte Thioestergruppe enthält, wobei R' ausgewählt ist aus der Gruppe bestehend aus Wasserstoff, einer gegebenenfalls geeignet substituierten linearen, cyclischen, und/oder verzweigten Alkyl-, Aryl-, Heteroaryl-, Aralkyl- und Heteroaralkylgruppe, bevorzugt Benzylgruppe, wobei X ausgewählt ist aus der Gruppe bestehend aus SH
und 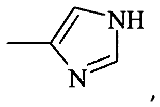 und wobei die Gruppe -(C=Y) aus der Thioestergruppe -(C=Y)-S-R' stammt und die Gruppe HN-CH-CH₂-X aus der alpha-X beta-Aminogruppe stammt, und wobei die funktionelle Gruppe M ausgewählt ist aus der Gruppe bestehend aus 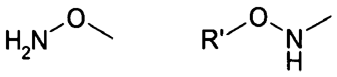 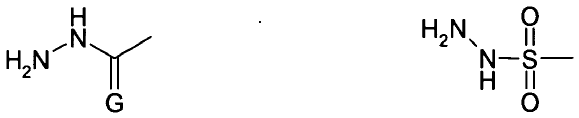 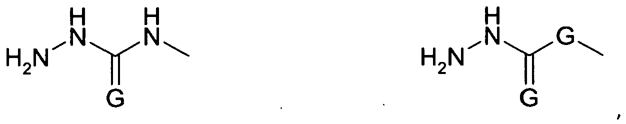 wobei G gleich O oder S und, falls zweimal vorhanden, unabhängig O oder S ist und R' Methyl ist.

2. Verfahren gemäß Anspruch 1, wobei die thioester-funktionalisierte Hydroxyalkylstärke mit einer alpha-X beta-Aminogruppe des Wirkstoffs umgesetzt wird, wobei die alpha-X beta-Aminogruppe in einem Cystein- oder Histidinrest des Wirkstoffes enthalten ist.

3. Verfahren gemäß Anspruch 1 oder 2, wobei die funktionelle Gruppe Q die chemische Struktur -NH- enthält.

4. Verfahren gemäß einem der Ansprüche 1 bis 3, wobei die funktionelle Gruppe Q ausgewählt ist aus der Gruppe bestehend aus
H₂N- 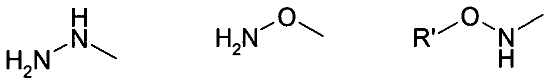 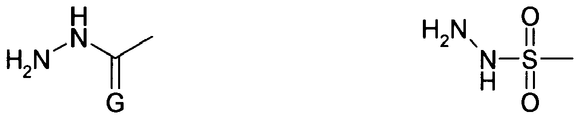 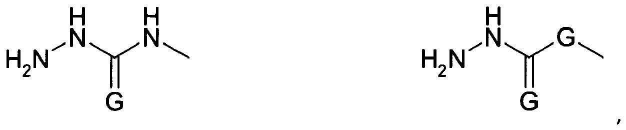
wobei G gleich O oder S und, falls zweimal vorhanden, unabhängig O oder S ist und R' Methyl ist.

5. Verfahren gemäß einem der Ansprüche 1 bis 4, wobei die funktionelle Gruppe Q eine Aminogruppe -NH₂ ist.

6. Konjugat aus einem Wirkstoff und Hydroxyalkylstärke, wobei der Wirkstoff und die Hydroxyalkylstärke durch eine chemische Gruppe einer Struktur gemäß Formel (IV) 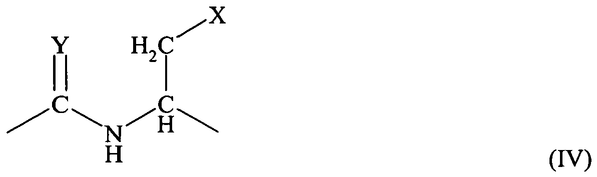 verbunden sind, wobei Y ein Heteroatom ist, ausgewählt aus der Gruppe bestehend aus O und S, und X ausgewählt ist aus der Gruppe bestehend aus SH und 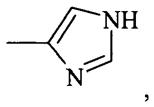 und das besagte Konjugat eine Struktur gemäß Formel (II) 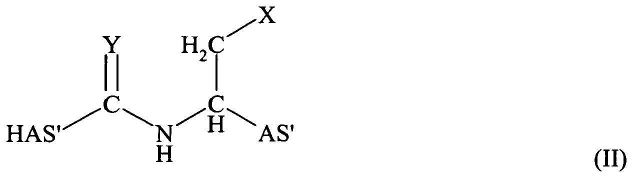 aufweist, wobei HAS' der Rest des Hydroxyalkylstärkederivats ist, das mit der Thioestergruppe verbunden wurde durch Umsetzen der Hydroxyalkylstärke mit einer zumindest bifunktionellen Verbindung enthaltend eine funktionelle Gruppe M, die mit der Hydroxyalkylstärke über das nicht-oxidierte reduzierende Ende umgesetzt wird, und eine funktionelle Gruppe Q, die eine Thioestergruppe oder eine funktionelle Gruppe ist, die weiter modifiziert wird, um eine Thioestergruppe zu erhalten, um das Hydroxyalkylstärkederivat zu erhalten, das besagte Thioestergruppe enthält, und wobei AS' der Rest des Wirkstoffs oder eines Derivats davon ist, welcher mit der alpha-X beta-Aminogruppe verbunden wurde, und wobei die funktionelle Gruppe M ausgewählt ist aus der Gruppe bestehend aus 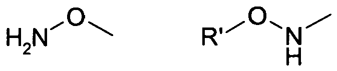 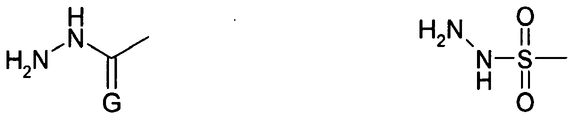 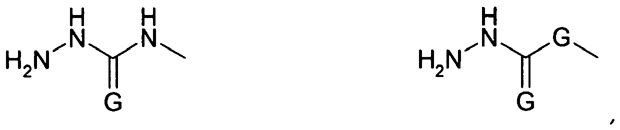 wobei G gleich O oder S und, falls zweimal vorhanden, unabhängig O oder S ist und R' Methyl ist, und wobei die Gruppe -(C=Y) aus der Thioestergruppe -(C=Y)-S-R' stammt und die Gruppe HN-CH-CH₂-X aus der alpha-X beta-Aminogruppe stammt.

7. Konjugat gemäß Anspruch 6, wobei der Wirkstoff ausgewählt ist aus der Gruppe bestehend aus Proteinen, Peptiden und niedermolekularen Wirkstoffen.

8. Konjugat gemäß den Ansprüchen 6 bis 7, wobei die Hydroxyalkylstärke Hydroxyethylstärke mit einem mittleren Molekulargewicht von 1 bis 300 kD, bevorzugt von 2 bis 200 kD, weiter bevorzugt von 4 bis 130 kD ist und einen Substitutionsgrad im Bereich von 0,1 bis 0,8 aufweist.

9. Konjugat gemäß einem der Ansprüche 6 bis 8 zur Verwendung in einem Verfahren zur Behandlung des menschlichen oder tierischen Körpers.

10. Pharmazeutische Zusammensetzung enthaltend ein Konjugat gemäß einem der Ansprüche 6 bis 8.

11. Pharmazeutische Zusammensetzung gemäß Anspruch 10, weiterhin enthaltend mindestens ein(en) pharmazeutisch verträgliches (verträglichen) Verdünnungsmittel, Hilfsstoff oder Träger.

12. Zusammensetzung, enthaltend ein Konjugat aus einem Wirkstoff und Hydroxyalkylstärke gemäß einem der Ansprüche 6 bis 8.

## Revendications

1. Procédé pour préparer un conjugué d'une substance active et d'hydroxyalkyl-amidon, dans lequel la substance active et l'hydroxyalkyl-amidon sont liés de façon covalente par un résidu chimique ayant une structure selon la formule (I) 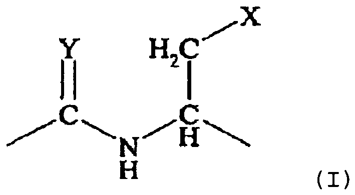 dans lequel Y est un hétéroatome, choisi dans le groupe constitué de O et S, ledit procédé comprenant la réaction d'un groupe thioester -(C=Y)-S-R' d'un dérivé d'hydroxyalkyl-amidon comprenant ledit groupe thioester avec un groupe alpha-X bêta-amino 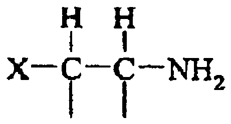 d'un dérivé de substance active comprenant ledit groupe alpha-X bêta-amino, ledit procédé comprenant en outre la réaction d'un hydroxyalkyl-amidon avec un composé au moins bifonctionnel comprenant un groupe fonctionnel M qui réagit avec l'hydroxyalkyl-amidon via l'extrémité réductrice non oxydée, et un groupe fonctionnel Q qui est un groupe thioester ou un groupe fonctionnel qui est en outre modifié pour obtenir un groupe thioester, pour obtenir le dérivé d'hydroxyalkyl-amidon comprenant ledit groupe thioester, dans lequel R' est choisi dans le groupe constitué de l'hydrogène, un groupe alkyle, aryle, hétéroaryle, aralkyle, et hétéroaralkyle linéaire, cyclique et/ou ramifié facultativement substitué de manière approprié, de préférence un benzyle,
dans lequel X est choisi dans le groupe constitué de SH
et 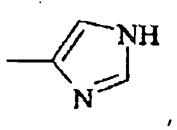 et dans lequel le groupe -(C=Y) est dérivé du groupe thioester -(C=Y)-S-R' et le groupe HN-CH-CH₂-X est dérivé du groupe alpha-X bêta-amino, et dans lequel le groupe fonctionnel M est choisi dans le groupe constitué de 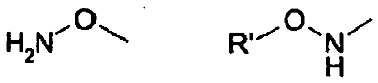 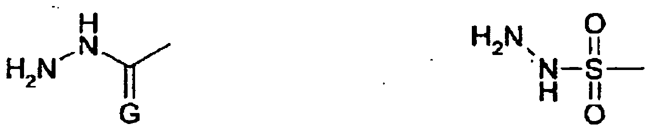 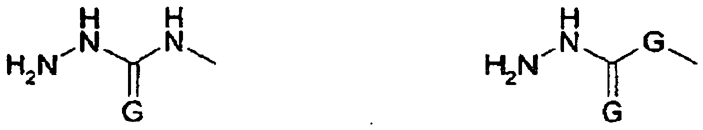 dans lequel G est O ou S et, s'il est présent deux fois, indépendamment O ou S, et R' est un méthyle.

2. Procédé selon la revendication 1, dans lequel l'hydroxyalkyl-amidon fonctionnalisé par thioester réagit avec un groupe alpha-X bêta-amino de la substance active dans lequel le groupe alpha-X bêta-amino est compris dans un résidu cystéine ou histidine de la substance active.

3. Procédé selon la revendication 1 ou 2, dans lequel le groupe fonctionnel Q comprend la structure chimique -NH-.

4. Procédé selon l'une quelconque des revendications 1 à 3, dans lequel le groupe fonctionnel Q est choisi dans le groupe constitué de
H₂N- 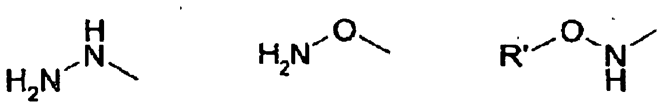 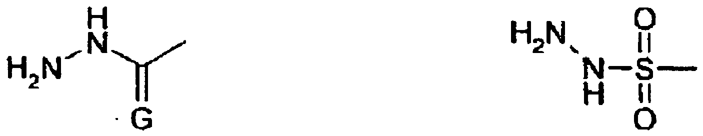 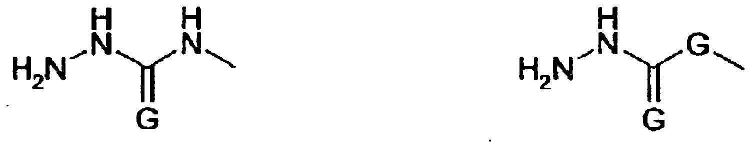
dans lequel G est O ou S et, s'il est présent deux fois, indépendamment O ou S, et R' est un méthyle.

5. Procédé selon l'une quelconque des revendications 1 à 4, dans lequel le groupe fonctionnel Q est un groupe amino -NH₂.

6. Conjugué d'une substance active et d'hydroxyalkyl-amidon, dans lequel la substance active et l'hydroxyalkyl-amidon sont liés par un fragment chimique, ayant une structure selon la formule (IV) 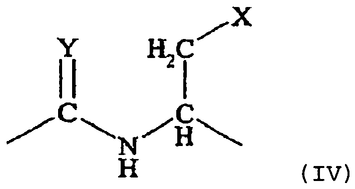 dans lequel Y est un hétéroatome, choisi dans le groupe constitué de O et S, et X est choisi dans le groupe
constitué de SH et 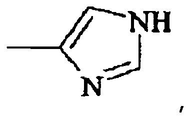 ledit conjugué ayant une structure selon la formule (II) 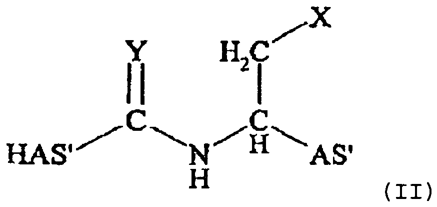 dans lequel HAS' est le résidu du dérivé d'hydroxyalkyl-amidon qui est lié au groupe thioester par réaction de l'hydroxyalkyl-amidon avec un composé au moins bifonctionnel comprenant un groupe fonctionnel M qui réagit avec l'hydroxyalkyl-amidon via l'extrémité réductrice non oxydée, et un groupe fonctionnel Q qui est un groupe thioester ou un groupe fonctionnel qui est en outre modifié pour obtenir un groupe thioester, pour obtenir le dérivé d'hydroxyalkyl-amidon comprenant ledit groupe thioester, et dans lequel AS' est le résidu de la substance active ou un dérivé de celui-ci qui est lié au groupe alpha-X bêta-amino, et dans lequel le groupe fonctionnel M est choisi dans le groupe constitué de 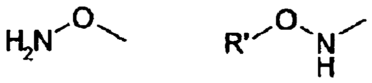 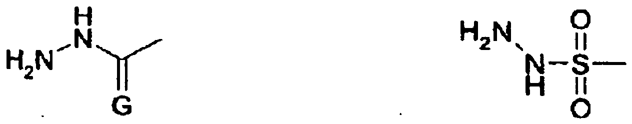 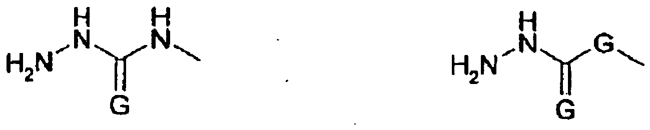 dans lequel G est O ou S et, s'il est présent deux fois, indépendamment O ou S, et R' est un méthyle, et dans lequel le groupe -(C=Y) est dérivé du groupe thioester -(C=Y)S-R' et le groupe HN-CH-CH₂-X est dérivé du groupe alpha-X bêta-amino.

7. Conjugué selon la revendication 6, dans lequel la substance active est choisie dans le groupe constitué de protéines, peptides, et médicaments à petite molécule.

8. Conjugué selon les revendications 6 et 7, dans lequel l'hydroxyalkyl-amidon est un hydroxyéthyl-amidon ayant un poids moléculaire moyen de 1 à 300 kD, de préférence de 2 à 200 kD, plus préférablement de 4 à 130 kD ; et un degré de substitution dans la plage de 0,1 à 0,8.

9. Conjugué selon l'une quelconque des revendications 6 à 8 pour utilisation dans un procédé de traitement du corps humain ou de l'animal.

10. Composition pharmaceutique comprenant un conjugué selon l'une quelconque des revendications 6 à 8.

11. Composition pharmaceutique selon la revendication 10, comprenant en outre au moins un diluant, adjuvant, ou véhicule pharmaceutiquement acceptable.

12. Composition comprenant un conjugué d'une substance active et d'hydroxyalkyl-amidon selon l'une quelconque des revendications 6 à 8.
